# EUROPEAN PATENT APPLICATION

(11) **EP 1 813 282 A1**
(43) Date of publication of application: **01.08.2007**
(21) Application number: 05782159.7
(22) Date of filing: 02.09.2005
(51) Int. Cl.: A61K 45/00, A61P 9/14, A61K 31/5377, A61K 31/496, A61K 31/454, A61K 31/4035

(54) **REMEDY FOR ANGIOSPASM ACCOMPANYING SUBARACHNOID HEMORRHAGE CONTIANING THROMBIN RECEPTOR ANTAGONIST AS THE ACTIVE INGREDIENT**

(30) Priority: 09.11.2004 US 626412 P; 15.03.2005 WO PCT/JP2005/005068
(71) Applicant: Eisai R&D Management Co., Ltd., Tokyo 112-8088 (JP); Kyushu University, National University Corporation, Higashi-ku Fukuoka-shi Fukuoka 812-8581 (JP)
(72) Inventor: HIRANO, Katsuya,, Fukuoka-shi, Fukuoka 812-8581 (JP); MAEDA, Yoshihisa, Fukuoka-shi, Fukuoka 812-8581 (JP); SASAKI, Tomio, Fukuoka-shi, Fukuoka 812-8581 (JP); KANAIDE, Hideo, Fukuoka-shi, Fukuoka 812-8581 (JP); KAI, Yasutoshi, Fukuoka-shi, Fukuoka 812-8581 (JP)
(74) Representative: Woods, Geoffrey Corlett
(86) International application number: PCT/JP2005/016568
(87) International publication number: WO 2006/051648

(57) **Abstract**

A therapeutic agent for subarachnoid hemorrhage or a drug for improving prognosis of subarachnoid hemorrhage, comprising a compound having a PAR1 inhibitory effect, its pharmaceutically acceptable salt or a hydrate thereof.

## Description

### FIELD OF THE INVENTION

The present invention relates to a therapeutic agent for subarachnoid hemorrhage.

### BACKGROUND OF THE INVENTION

Subarachnoid hemorrhage (SAH) is a disease that accounts for 10% of the cerebral strokes. Patients who suffer from subarachnoid hemorrhage may face death in a serious case or even if the patients survive they often suffer from severe disorder.

Subarachnoid hemorrhage is a disease that is caused by bleeding in the cerebrospinal fluid space between the thin membrane surrounding the brain (arachnoid membrane) and the brain. Since subarachnoid hemorrhage is bleeding caused on the surface of the brain rather than inside the brain parenchyma, neurological symptoms caused by cerebral vasospasm are more problem than neurological symptoms caused by brain compression or brain necrosis associated with the bleeding. Accordingly, cerebral vasospasm accompanied by bleeding is believed to be one of the key elements that determine the prognosis of subarachnoid hemorrhage.

The molecular mechanism upon incidence of cerebral vasospasm, however, has not yet been solved and there is a need for understanding this mechanism for treating subarachnoid hemorrhage.

Meanwhile, thrombin is one of the blood coagulation factors. Thrombin is known to activate protease-activated receptor-1 (PAR1) and shows regulatory activity on vascular contraction.

So far, relationship between cerebral vasospasm associated with subarachnoid hemorrhage and thrombin and PAR1 has not been studied in detail. Also, remedies for subarachnoid hemorrhage that utilize a mechanism relating to thrombin and PAR1 have not been studied.

### DISCLOSURE OF THE INVENTION

The present invention provides a drug effective for cerebral vasospasm associated with subarachnoid hemorrhage.

So far, administrations of anti-thrombin III that inhibits thrombin activity and synthetic serine protease inhibitor FUT-175 to a subarachnoid hemorrhage animal model have been reported to suppress angiospasm (Tsuratani et al., Stroke, 34:1497-1500, 2003; Yanamoto et al., Nerosurgery, 30:358-363, 1992). Inhibition of thrombin, however, may cause further bleeding and thus thrombin inhibitors have been contemplated difficult to be used as remedies for subarachnoid hemorrhage.

The present inventors have devoted a significant amount of research effort to solving the above-mentioned problems, as a result of which they found that the cerebral blood vessel showed a high contractile property as mediated by protease-activated receptor-1 (PAR1), that upregulation of PAR1 was induced and that desensitization of PAR1 was deteriorated in a subarachnoid hemorrhage animal model. Since PAR1-mediated stimulation causes, in addition to triggering cerebral vasospasm following subarachnoid hemorrhage, induction and impaired desensitization of PAR1, inhibition of PAR1 was considered to have chances of not only regressing cerebral vasospasm following subarachnoid hemorrhage but also preventing occurrence of subarachnoid hemorrhage.

Based on the findings above, the present inventors have demonstrated that it is possible to treat subarachnoid hemorrhage by inhibition of PAR1 without causing further bleeding, thereby completed the present invention. Thus, the present invention is as follows.
(1) A therapeutic agent for subarachnoid hemorrhage or a drug for improving prognosis of subarachnoid hemorrhage, comprising a compound having an effect of inhibiting the function of protease-activated receptor-1, its pharmaceutically acceptable salt or a hydrate thereof.
(2) A therapeutic agent or an improving drug according to (1), wherein the compound having the effect of inhibiting the function of protease-activated receptor-1 is an antagonist of protease-activated receptor-1.
(3) A therapeutic agent or an improving drug according to either one of (1) and (2),
   wherein the compound having the effect of inhibiting the function of protease-activated receptor-1 is 2-iminopyrrolidine derivative.
(4) A therapeutic agent for subarachnoid hemorrhage or a drug for improving prognosis of subarachnoid hemorrhage comprising a 2-iminopyrrolidine derivative,
   wherein the 2-iminopyrrolidine derivative is a compound represented by Formula (I) 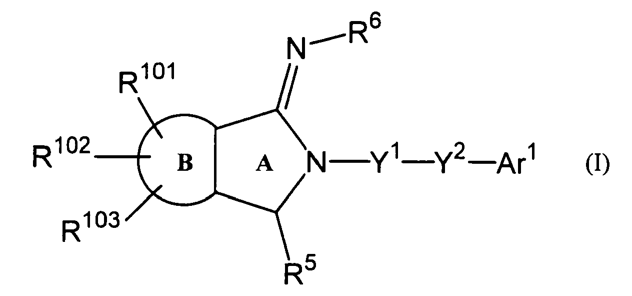 [wherein, ring A indicates a pyrrolidine ring; ring B indicates a benzene ring or a pyridine ring; R¹⁰¹, R¹⁰² and R¹⁰³ independently indicate, identically or differently, a hydrogen atom, a halogen atom, C₁-C₆ alkyl group or C₁-C₆ alkoxy group; R⁵ indicates a hydrogen atom, C₁-C₆ alkyl group or C₁-C₆ alkoxy C₁-C₆ alkyl group; R⁶ indicates a hydrogen atom, C₁-C₆ alkyl group or C₁-C₆ alkyloxy carbonyl group; Y¹ indicates a single bond or -CH₂-; Y² indicates a single bond or -CO-; Ar¹ indicates a hydrogen atom or a group represented by Formula (II) 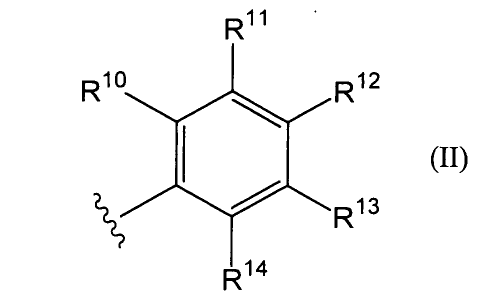 (wherein, R¹⁰, R¹¹, R¹², R¹³ and R¹⁴ independently indicate, identically or differently, a hydrogen atom, C₁-C₆ alkyl group, hydroxyl group, C₁-C₆ alkoxy group, morpholinyl group, piperazinyl group which may or may not have a substituent, piperidinyl group which may or may not have a substituent or pyrrolidinyl group which may or may not have a substituent, and R¹¹ and R¹² or R¹² and R¹³ may bind to each other to form a 5-8-membered heterocycle)], its pharmaceutically acceptable salt, or a hydrate thereof.
(5) A therapeutic agent for subarachnoid hemorrhage or a drug for improving prognosis of subarachnoid hemorrhage comprising a 2-iminopyrrolidine derivative,
   wherein the 2-iminopyrrolidine derivative is a compound represented by Formula (III) 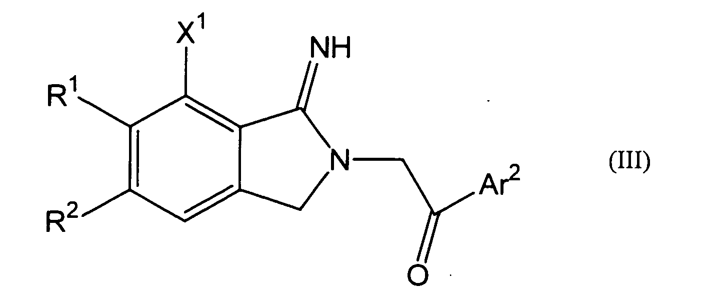 [wherein, R¹ and R² independently indicate, identically or differently, a hydrogen atom, methoxy group or ethoxy group; X¹ indicates a hydrogen atom or a halogen atom; Ar² indicates methyl group, ethyl group, methoxy group, ethoxy group, t-butyl group, morpholinyl group or phenyl group which may be substituted with one or more substituents selected from substituents represented by the following Formula (IV), 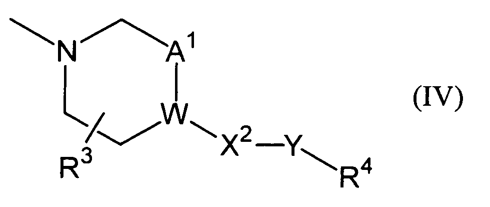 wherein, W indicates -CH- or a nitrogen atom; A¹ indicates -CH₂- or a single bond; R³ indicates a hydrogen atom or -OR^{5a}; X² indicates -CH₂-, an oxygen atom, a single bond or carbonyl group; Y indicates a single bond or C₁-C₄ alkylene group; R⁴ indicates a hydrogen atom, -OR^{6a}, cyano group or -COOR⁷; R^{5a}, R^{6a} and R⁷ independently indicate, identically or differently, a hydrogen atom or C₁-C₄ alkyl group], its pharmaceutically acceptable salt or a hydrate thereof.
(6) A therapeutic agent or an improving drug according to (5) wherein R¹ and R² are ethoxy groups while X¹ is a fluorine atom.
(7) A therapeutic agent for subarachnoid hemorrhage or a drug for improving prognosis of subarachnoid hemorrhage comprising a 2-iminopyrrolidine derivative,
   wherein the 2-iminopyrrolidine derivative is one selected from the group consisting of compounds represented by Formulae (V)-(XI), its pharmaceutically acceptable salt or a hydrate thereof. 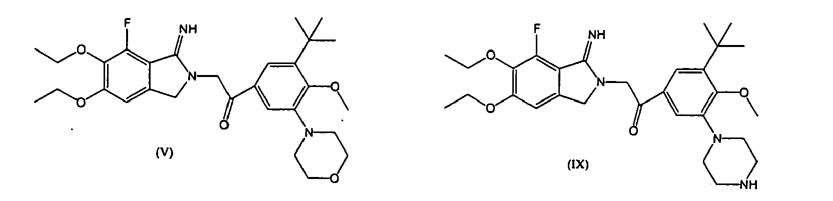 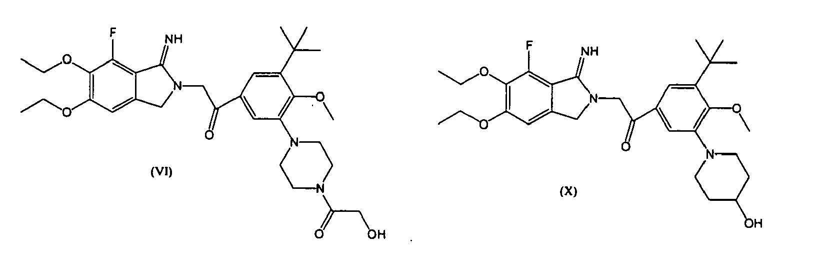 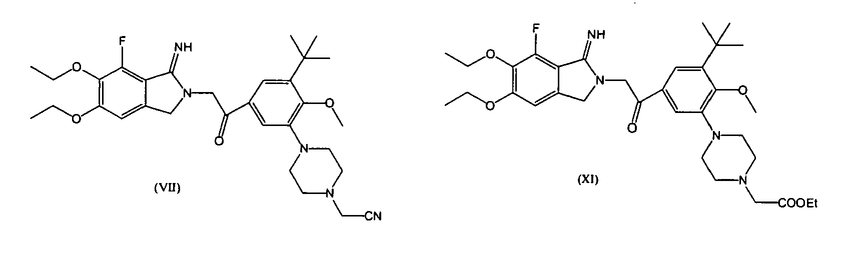 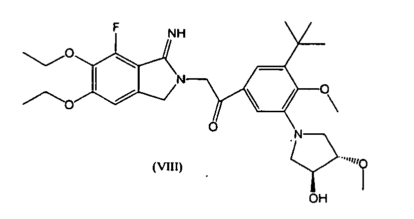
(8) A therapeutic agent for subarachnoid hemorrhage or a drug for improving prognosis of subarachnoid hemorrhage comprising a 2-iminopyrrolidine derivative, wherein the 2-iminopyrrolidine derivative is a compound represented by Formula (V), its pharmaceutically acceptable salt or a hydrate thereof. 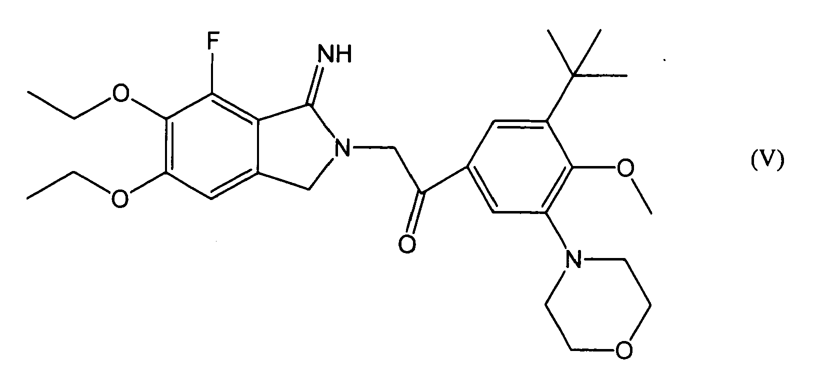
(9) An antivasospastic agent comprising a compound having an effect of inhibiting the function of protease-activated receptor-1, its pharmaceutically acceptable salt or a hydrate thereof.
(10) An antivasospastic agent according to (9), wherein the compound having the effect of inhibiting the function of protease-activated receptor-1 is an antagonist of protease-activated receptor-1.
(11) An antivasospastic agent according to either one of (9) and (10) wherein the compound having the effect of inhibiting the function of protease-activated receptor-1 is a 2-iminopyrrolidine derivative.
(12) An antivasospastic agent comprising a 2-iminopyrrolidine derivative, wherein the 2-iminopyrrolidine derivative is a compound represented by Formula (I) 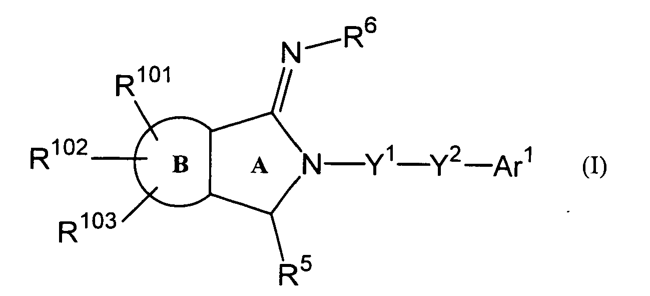 [wherein, ring A indicates a pyrrolidine ring; ring B indicates a benzene ring or a pyridine ring; R¹⁰¹, R¹⁰² and R¹⁰³ independently indicate, identically or differently, a hydrogen atom, a halogen atom, C₁-C₆ alkyl group or C₁-C₆ alkoxy group; R⁵ indicates a hydrogen atom, C₁-C₆ alkyl group or C₁-C₆ alkoxy C₁-C₆ alkyl group; R⁶ indicates a hydrogen atom, C₁-C₆ alkyl group or C₁-C₆ alkyloxy carbonyl group; Y¹ indicates a single bond or -CH₂-; Y² indicates a single bond or -CO-; Ar¹ indicates a hydrogen atom or a group represented by Formula (II) 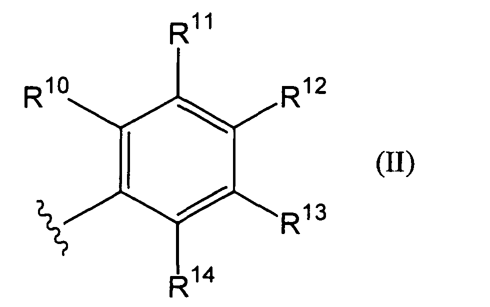 (wherein, R¹⁰, R¹¹, R¹², R¹³ and R¹⁴ independently indicate, identically or differently, a hydrogen atom, C₁-C₆ alkyl group, hydroxyl group, C₁-C₆ alkoxy group, morpholinyl group, piperazinyl group which may or may not have a substituent, piperidinyl group which may or may not have a substituent or pyrrolidinyl group which may or may not have a substituent, and R¹¹ and R¹² or R¹² and R¹³ may bind to each other to form a 5-8-membered heterocycle)], its pharmaceutically acceptable salt or a hydrate thereof.
(13) An antivasospastic agent comprising a 2-iminopyrrolidine derivative, wherein the 2-iminopyrrolidine derivative is a compound represented by Formula (III) 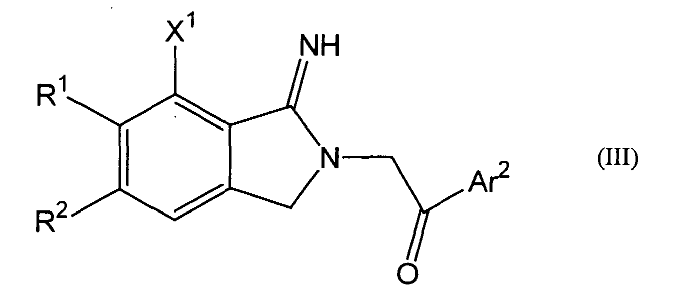 [wherein, R¹ and R² independently indicate, identically or differently, a hydrogen atom, methoxy group or ethoxy group; X¹ indicates a hydrogen atom or a halogen atom; Ar² indicates methyl group, ethyl group, methoxy group, ethoxy group, t-butyl group, morpholinyl group or phenyl group which may be substituted with one or more substituents selected from substituents represented by the following Formula (IV), 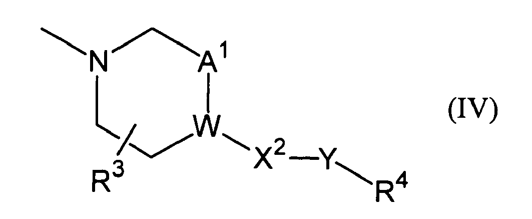 (wherein, W indicates -CH- or a nitrogen atom; A¹ indicates -CH₂- or a single bond; R³ indicates a hydrogen atom or -OR^{5a}; X² indicates -CH₂-, an oxygen atom, a single bond or carbonyl group; Y indicates a single bond or C₁-C₄ alkylene group; R⁴ indicates a hydrogen atom, -OR^{6a}, cyano group or -COOR⁷; R^{5a}, R^{6a} and R⁷ independently indicate, identically or differently, a hydrogen atom or C₁-C₄ alkyl group)], its pharmaceutically acceptable salt or a hydrate thereof.
(14) An antivasospastic agent according to (13), wherein R¹ and R² are ethoxy groups while X¹ is a fluorine atom.
(15) An antivasospastic agent comprising a 2-iminopyrrolidine derivative, wherein the 2-iminopyrrolidine derivative is any one selected from the group consisting of compounds represented by Formulae (V)-(XI), its pharmaceutically acceptable salt or a hydrate thereof. 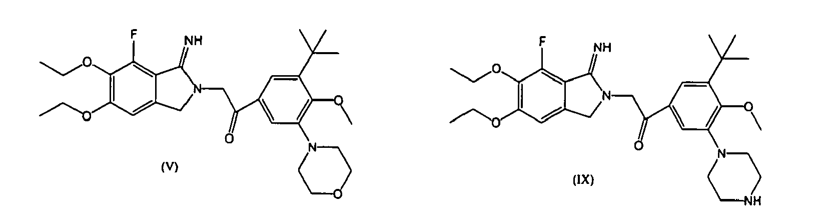 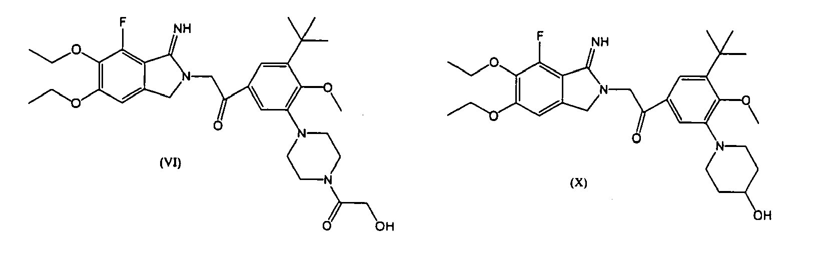 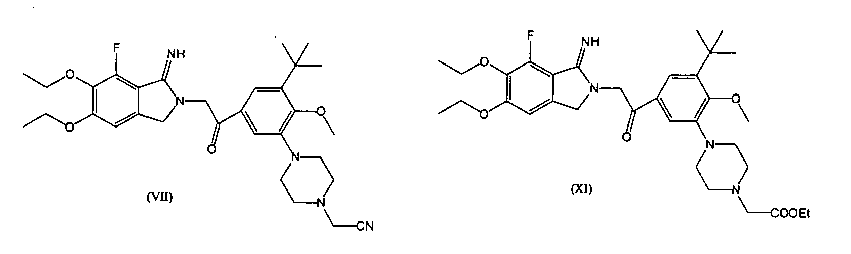 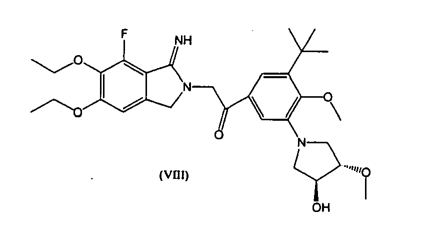
(16) An antivasospastic agent comprising a 2-iminopyrrolidine derivative, wherein the 2-iminopyrrolidine derivative is a compound represented by Formula (V), its pharmaceutically acceptable salt or a hydrate thereof. 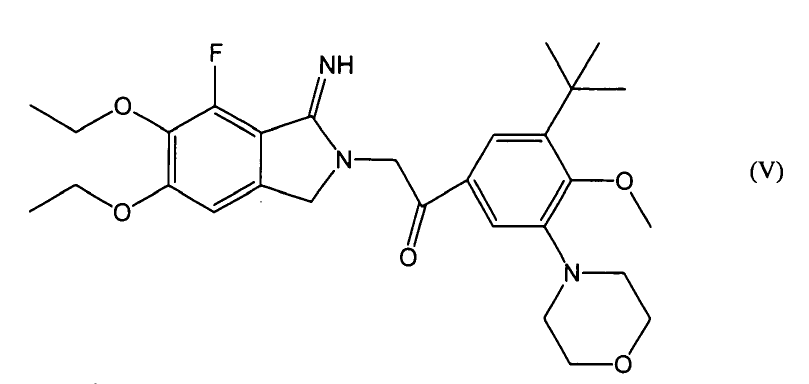
(17) A method for treating subarachnoid hemorrhage or a method for improving prognosis of subarachnoid hemorrhage, comprising administering an effective amount of a compound having an effect of inhibiting the function of protease-activated receptor-1, its pharmaceutically acceptable salt or a hydrate thereof to a patient.
(18) A method for treating subarachnoid hemorrhage or a method for improving prognosis of subarachnoid hemorrhage, comprising administering an effective amount of the 2-iminopyrrolidine derivative according to at least one of (4)-(8) to a patient.
(19) A method for preventing angiospasm, comprising administering an effective amount of a compound having an effect of inhibiting the function of protease-activated receptor-1, its pharmaceutically acceptable salt or a hydrate thereof to a patient.
(20) A method for preventing angiospasm, comprising administering an effective amount of the 2-iminopyrrolidine derivative according to at least one of (12)-(16) to a patient.
(21) Use of the 2-iminopyrrolidine derivative according to at least one of (4)-(8) for producing a therapeutic agent for subarachnoid hemorrhage or a drug for improving prognosis of subarachnoid hemorrhage.
(22) Use of the 2-iminopyrrolidine derivative according to at least one of (12)-(16) for producing an antivasospastic agent.

### BRIEF DESCRIPTION OF THE DRAWINGS

Figure 1 is a view showing PAR1 activation mechanisms by thrombin and PAR1-activating peptide (PAR1-AP).
Figure 2 is a view showing an experimental procedure for rabbit double-hemorrhage models.
Figure 3 is a view showing contraction induced by hyperkalemic depolarization and contraction induced by endothelin-1 in endothelium-removed basilar arteries.
Figure 4 is a view showing contractile responses of rabbit basilar arteries to thrombin for the control group and the SAH group.
Figure 5 is a view showing contractile responses of rabbit basilar arteries to PAR1-AP for the control group and the SAH group.
Figure 6 is a view showing inhibitory activities of heparinized autologous bloods on enhanced contractile effect in the SAH group.
Figure 7 is a view showing contractile responses of α toxin-skinned basilar arteries induced by increase in calcium ion (Ca²⁺) concentration and GTPγS.
Figure 8 is a view showing contractile responses of α toxin-skinned basilar arteries to thrombin and PAR1-AP for the control group and the SAH group.
Figure 9 is a view showing changes in upregulation of PAR1 mRNA with time for the SAH group.
Figure 10 is a view showing an effect of 100 µM PAR1-AP on prolonged response for the SAH group.
Figure 11 is a view showing irreversible contraction by thrombin in the SAH group.
Figure 12 is a view showing an effect of PAR1 antagonist on contractile responses to thrombin.
Figure 13 is a view showing an effect of PAR1 antagonist on contractile responses to thrombin.

### BEST MODES FOR CARRYING OUT THE INVENTION

Hereinafter, the present invention will be described in detail. The following embodiments are illustration of the present invention and are not intended to limit the invention. The present invention may be carried out in various embodiments as long as they do not depart from the scope of the invention.

Publications cited herein are incorporated herein in their entirety.

Based on the new findings that cerebral vasospasm is induced when PAR1 is upregulated and desensitization of PAR1 is impaired in a patient with subarachnoid hemorrhage, the present invention was accomplished by finding that a compound having an effect of inhibiting the enhanced PAR1 function is effective in treating subarachnoid hemorrhage. Thus, the present invention provides a therapeutic agent or a prognosis improving drug for subarachnoid hemorrhage or an antivasospastic agent, comprising a compound that inhibits PAR1 function to suppress cerebral vasospasm (i.e., a PAR1 inhibitor) as an active ingredient. Furthermore, the present invention provides a method for treating subarachnoid hemorrhage, a method for improving prognosis of subarachnoid hemorrhage or a method for preventing angiospasm comprising administering an effective amount of a compound having an effect of inhibiting the function of PAR1 to a patient. A blood vessel is preferably a blood vessel of brain, more preferably a cerebral vessel.

### 1. Compound having effect of inhibiting function of PAR1

### (1) PAR1

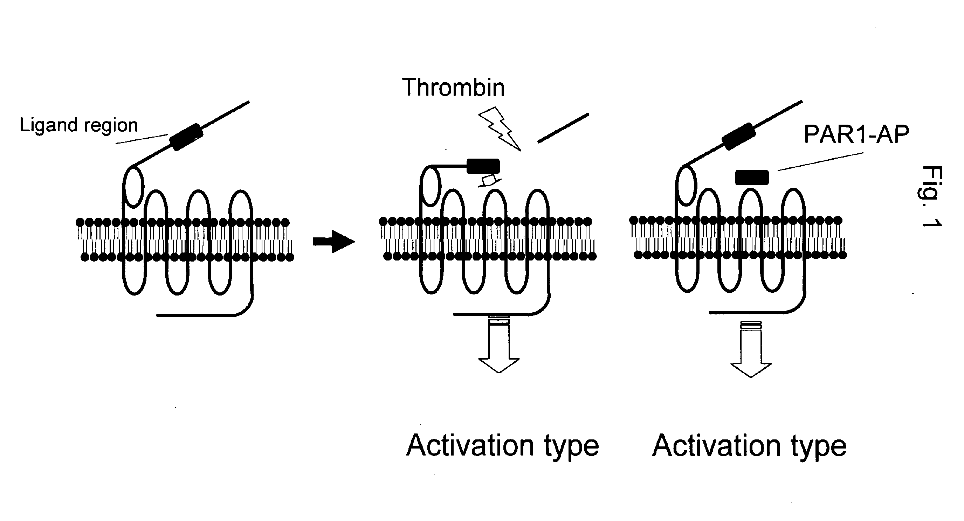

PAR1 is one of protease-activated receptors, and is a G-protein-coupled receptor that is activated due to degradation of a particular extracellular region by protease. The activation mechanism of PAR1 is shown in Figure 1. Activation of the receptor occurs when a certain N-terminal site of the receptor is cleaved with serine protease to expose the receptor-activating sequence, which acts as a ligand and binds to the ligand binding site of the receptor. As an agonist of PAR1, other than thrombin and trypsin that act as proteases, a synthesized peptide of a receptor-activating sequence (e.g., PAR1-AP (PAR1 activating peptide)) is also known to act as an agonist. As such a PAR1-AP sequence, SFLLRN (human, single-letter amino acid code, SEQ ID NO:1), TFRIFD (frog, single-letter amino acid code, SEQ ID NO:2) and the like have been identified.

### (2) Compound having effect of inhibiting function of PAR1

As used herein, "a compound having an effect of inhibiting the function of PAR1" (also referred to as a "PAR1 inhibitor" in this specification) is not limited as long as it is a substance that has an effect of suppressing PAR1 activation, and refers to PAR1-desensitization promoting agents, PAR1 antisense oligonucleotides, PAR1 siRNA, PAR1-neutralizing antibody or the like including PAR1 antagonists. Examples of preferable properties of the PAR1 inhibitor used with the invention include that it has an effect of suppressing cerebral vasospasm, that it is highly selective of PAR1, that it arises centrally, that it does not cause severe side-effects (such as further bleeding) at a therapeutically effective amount and that it has an immediate effect. In addition, a PAR1 inhibitor according to the present invention comprises its pharmaceutically acceptable salt or a hydrate thereof (for details, see below).

Thus, according to the present invention, examples of a preferable compound to be used as a therapeutic agent or a prognosis improving drug for subarachnoid hemorrhage or an antivasospastic agent include a "compound having an effect of inhibiting the function of PAR1", particularly a PAR antagonist, its pharmaceutically acceptable salt and a hydrate thereof.

As used herein, a "PAR1 antagonist" or an "antagonist of PAR1" refers to a substance that binds to PAR1 and that inhibits binding between a polypeptide part containing a receptor-activating sequence and PAR1 (so-called PAR1 antagonist

### (3) 2-Iminopyrrolidine derivative

As a PAR1 antagonist used with the present invention, a 2-iminopyrrolidine derivative may be used.

According to the present invention, a 2-iminopyrrolidine derivative includes a compound represented by the following Formula (I), its pharmaceutically acceptable salt or a hydrate thereof. 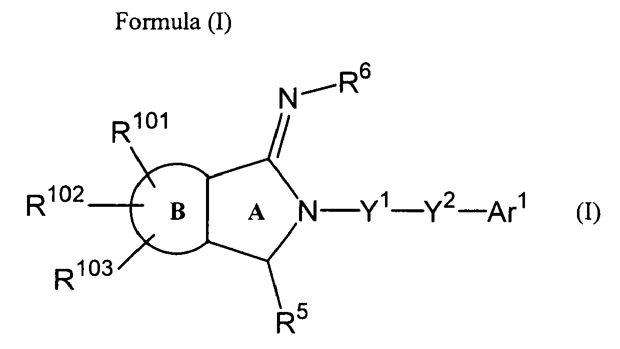 wherein,
ring A indicates a pyrrolidine ring;
ring B indicates a benzene ring or a pyridine ring;
R¹⁰¹, R¹⁰² and R¹⁰³ independently indicate, identically or differently, a hydrogen atom, a halogen atom, C₁-C₆ alkyl group or C₁-C₆ alkoxy group;
R⁵ indicates a hydrogen atom, C₁-C₆ alkyl group or C₁-C₆ alkoxy C₁-C₆ alkyl group;
R⁶ indicates a hydrogen atom, C₁-C₆ alkyl group or C₁-C₆ alkyloxy carbonyl group;
Y¹ indicates a single bond or -CH₂-;
Y² indicates a single bond or -CO-;
Ar¹ indicates a hydrogen atom or a group represented by the following Formula (II): 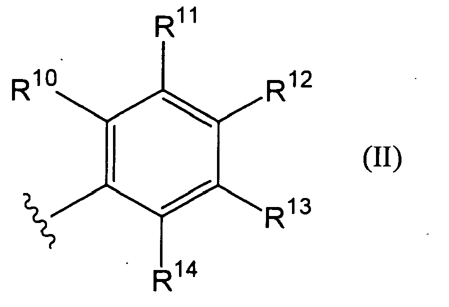 wherein,
R¹⁰, R¹¹, R¹², R¹³ and R¹⁴ independently indicate, identically or differently, a hydrogen atom, C₁-C₆ alkyl group, hydroxyl group, C₁-C₆ alkoxy group, morpholinyl group, piperazinyl group which may or may not have a substituent, piperidinyl group which may or may not have a substituent or pyrrolidinyl group which may or may not have a substituent, and
further, R¹¹ and R¹² or R¹² and R¹³ may bind to each other to form a 5-8-membered heterocycle.

According to the present invention, substituents which piperazinyl group, piperidinyl group or pyrrolidinyl group may have include but not limited to one or more from the group consisting of, for example, hydroxyl group, cyanomethyl group, methoxy group, -COCH₂OH and -CH₂COOCH₂CH₃.

Moreover, according to the present invention, a 2-iminopyrrolidine derivative comprises a compound represented by the following Formula (III), its pharmaceutically acceptable salt or a hydrate thereof. 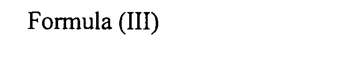 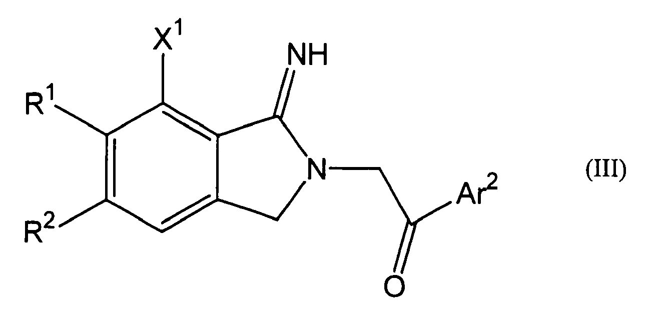 wherein,
R¹ and R² independently indicate, identically or differently, a hydrogen atom, methoxy group or ethoxy group;
X¹ indicates a hydrogen atom or a halogen atom;
Ar² indicates methyl group, ethyl group, methoxy group, ethoxy group, t-butyl group, morpholinyl group or phenyl group which may be substituted with one or more substituents selected from substituents represented by the following Formula (IV), 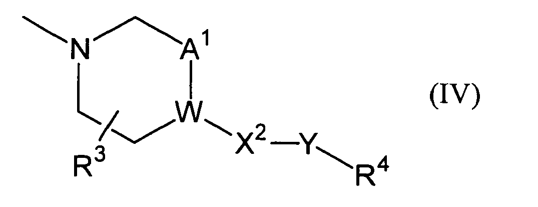 wherein,
W indicates -CH- or a nitrogen atom;
A¹ indicates -CH₂- or a single bond;
R³ indicates a hydrogen atom or -OR^{5a};
X² indicates -CH₂-, an oxygen atom, a single bond or carbonyl group;
Y indicates a single bond or C₁-C₄ alkylene group;
R⁴ indicates a hydrogen atom, -OR^{6a}, cyano group or -COOR⁷;
R^{5a} , R^{6a} and R⁷ independently indicate, identically or differently, a hydrogen atom or C₁-C₄ alkyl group.

Preferably, R¹ and R² in Formula (III) are ethoxy groups while X¹ is a fluorine atom.

As used herein, a "halogen atom" includes atoms such as a fluorine atom, a chlorine atom, a bromine atom, an iodine atom, and preferably a fluorine atom, a chlorine atom or a bromine atom.

As used herein, "C₁-C₆ alkyl group" refers to alkyl group with a carbon number of 1 to 6, preferable groups being, for example, linear or branched alkyl groups such as methyl group, ethyl group, n-propyl group, iso-propyl group, n-butyl group, iso-butyl group, sec-butyl group, tert-butyl group, n-pentyl group, 1,1-dimethylpropyl group, 1,2-dimethylpropyl group, 2,2-dimethylpropyl group, 1-ethylpropyl group, 2-ethylpropyl group, 1-methylbutyl group, 2-methylbutyl group, n-hexyl group, 1-methyl-2-ethylpropyl group, 1-ethyl-2-methylpropyl group, 1,1,2-trimethylpropyl group, 1-propylpropyl group, 1,1-dimethylbutyl group, 1,2-dimethylbutyl group, 2,2-dimethylbutyl group, 1,3-dimethylbutyl group, 2,3-dimethylbutyl group, 2-ethylbutyl group, 2-methylpentyl group, 3-methylpentyl group and the like, and more preferable groups being methyl group, ethyl group, n-propyl group, iso-propyl group, n-butyl group, iso-butyl group, sec-butyl group, tert-butyl group, n-pentyl group and the like.

As used herein, "C₁-C₄ alkyl group" refers to alkyl group with a carbon number of 1 to 4, preferable groups being, for example, linear or branched alkyl groups such as methyl group, ethyl group, n-propyl group, iso-propyl group, n-butyl group, iso-butyl group, sec-butyl group, tert-butyl group and the like, and more preferable groups being methyl group, ethyl group, n-propyl group, iso-propyl group, n-butyl group, iso-butyl group, sec-butyl group, tert-butyl group and the like.

As used herein, "C₁-C₆ alkoxy group" refers to alkoxy group with a carbon number of 1 to 6, preferable groups being, for example, methoxy group, ethoxy group, n-propoxy group, iso-propoxy group, sec-propoxy group, n-butoxy group, iso-butoxy group, sec-butoxy group, tert-butoxy group, n-pentyloxy group, iso-pentyloxy group, sec-pentyloxy group, 1,1-dimethylpropyloxy group, 1,2-dimethylpropoxy group, 2,2-dimethylpropyloxy group, n-hexoxy group, 1-ethylpropoxy group, 2-ethylpropoxy group, 1-methylbutoxy group, 2-methylbutoxy group, iso-hexoxy group, 1-methyl-2-ethylpropoxy group, 1-ethyl-2-methylpropoxy group, 1,1,2-trimethylpropoxy group, 1,1,2-trimethylpropoxy group, 1-propylpropoxy group, 1,1-dimethylbutoxy group, 1,2-dimethylbutoxy group, 2,2-dimethylbutoxy group, 2,3-dimethylbutyloxy group, 1,3-dimethylbutyloxy group, 2-ethylbutoxy group, 1,3-dimethylbutoxy group, 2-methylpentoxy group, 3-methylpentoxy group, hexyloxy group and the like.

As used herein, "C₁-C₆ alkoxy C₁-C₆ alkyl group" means C₁-C₆ alkyl group substituted with C₁-C₆ alkoxy group.

Furthermore, as used herein, a phrase "which may or may not have a substituent" is equivalent to "which may have one or more substituents in any combination at a replaceable position".

As used herein, "n-" refers to a normal or primary substituent, "sec-" refers to a secondary substituent, "t-(tert-)" refers to a tertiary substituent, and "i-(iso-)" refers to an isotype substituent.

Preferably, Formulae (I) and (III) of the invention comprise a compound represented by (V)-(XI), its pharmaceutically acceptable salt or a hydrate thereof, and any combination of one or more of these compounds may be used.

A compound that inhibits the function of PAR1, its pharmaceutically acceptable salt or a hydrate thereof used with the present invention may be produced by those skilled in the art according to a known method. 2-iminopyrrolidine derivatives represented by Formulae (I) and (III) can be produced according to the method described in WO 02/085855 pamphlet.

A preferable compound in Formulae (I) and (III) is a compound represented by Formulae (V)-(XI), its pharmaceutically acceptable salt or a hydrate thereof, more preferably a compound represented by Formula (V), its pharmaceutically acceptable salt or a hydrate thereof.

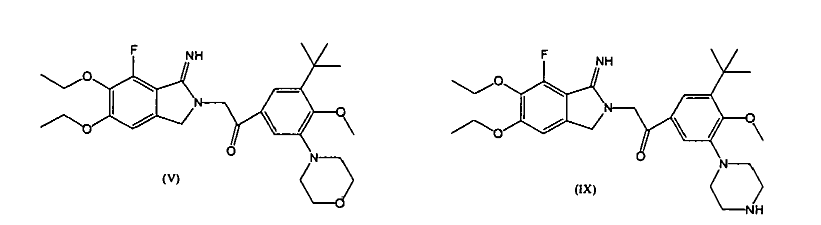

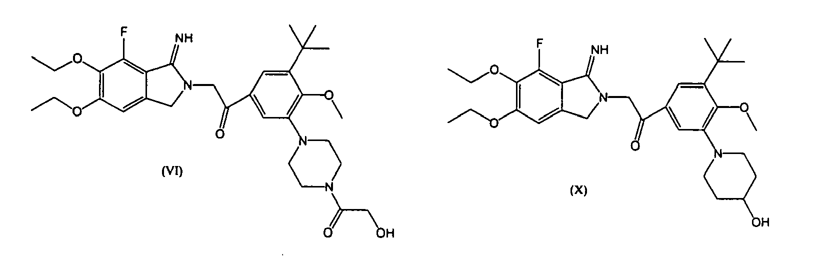

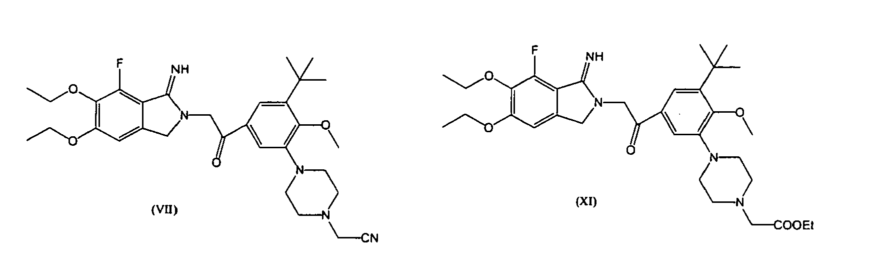

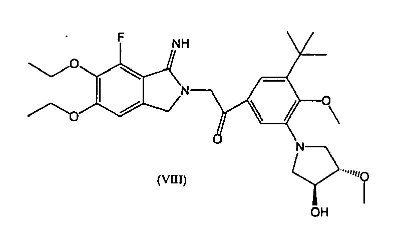

Herein, a compound represented by Formula (V) may also be referred to as "E5555".

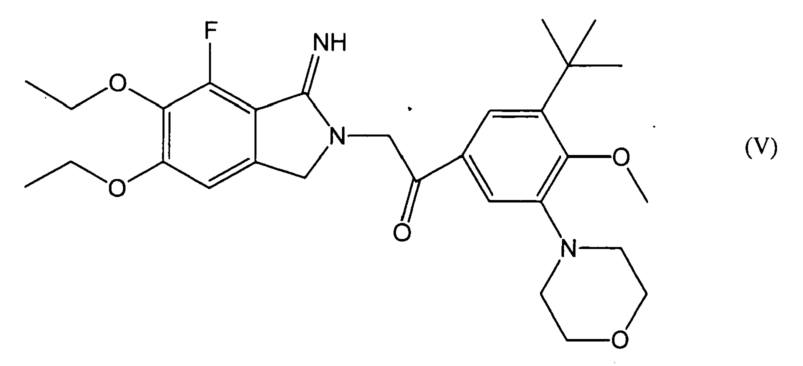

The 2-iminopyrrolidine derivatives represented by Formulae (V)-(XI) can be produced according to WO02/085855 pamphlet.

According to the present invention, a 2-iminopyrrolidine derivative may also form a salt with acid or base. Such a compound of the invention also comprises a pharmaceutically acceptable salt thereof. Herein, a salt means a "pharmaceutically acceptable salt", and such a pharmaceutically acceptable salt is not limited as long as it has an effect of inhibiting the function of PAR1 and forms a pharmaceutically acceptable salt with a compound of the invention as a therapeutic agent for subarachnoid hemorrhage. Specifically, examples include but not limited to hydrohalide (e.g., hydrofluoride, hydrochloride, hydrobromate, hydroiodide, etc.), salts of inorganic acids (e.g., sulfate, nitrate, perchlorate, phosphate, carbonate, bicarbonate, etc.), organic carboxylates (e.g., acetate, oxalate, maleate, tartrate, fumarate, citrate, etc.), organic sulfonates (e.g., methanesulfonate, trifluoromethanesulfonate, ethanesulfonate, benzenesulfonate, toluenesulfonate, camphorsulfonate, etc.), amino acid salts (e.g., aspartate, glutamate, etc.), quaternary amine salts, alkali metal salts (e.g., sodium salt, potassium salt, etc.) and alkaline earth metal salts (e.g., magnesium salt, calcium salt, etc.).

Moreover, according to the present invention, a 2-iminopyrrolidine derivative may have an asymmetric carbon depending on the type of the substituent, and may exist as an enantiomer such as a geometric isomer, an enantiomer and a diastereomer. These enantiomers are also included in the compound having the effect of inhibiting the function of PAR1 of the invention.

According to the present invention, a hydrate of the 2-iminopyrrolidine derivative, if any, is also included in the compound having the effect of inhibiting the function of PAR1 used with the invention.

### 2. Therapeutic agent and prognosis improving drug for subarachnoid hemorrhage, and antivasospastic agent

A pharmaceutical composition of the invention, namely a therapeutic agent or a prognosis improving drug for subarachnoid hemorrhage or an antivasospastic agent of the invention comprises a compound that has an effect of inhibiting the function of PAR1. The compound having the effect of inhibiting the function of PAR1 in the therapeutic agent, the improving drug and the antivasospastic agent of the invention is preferably a PAR1 antagonist, more preferably a 2-iminopyrrolidine derivative represented by Formula (I) or (III), still more preferably at least one compound selected from the compounds represented by Formulae (V)-(XI), and most preferably a compound represented by Formula (V). The PAR1 inhibitor contained in the therapeutic agent or the prognosis improving drug for subarachnoid hemorrhage or the antivasospastic agent of the invention comprises its pharmaceutically acceptable salt or a hydrate thereof.

A PAR1 inhibitor contained in the pharmaceutical composition of the invention has an effect of inhibiting the function of upregulated PAR1 or PAR1 with impaired desensitization mechanism. Thus, the pharmaceutical composition of the invention is effective in improving cerebral vasospasm associated with subarachnoid hemorrhage. Since cerebral vasospasm is one of the factors that determine prognosis of subarachnoid hemorrhage, the pharmaceutical composition of the invention that contains a PAR1 inhibitor may be used as a therapeutic agent or a prognosis improving drug for subarachnoid hemorrhage or as an antivasospastic agent.

Since the PAR1 inhibitor, its pharmaceutically acceptable salt and a hydrate thereof described above have the effect of inhibiting the function of PAR1, they are useful as an active ingredient of the therapeutic agent or the prognosis improving drug for subarachnoid hemorrhage or the antivasospastic agent of the invention.

Herein, an "antivasospastic agent" means a pharmaceutical composition that prevents, suppresses and/or reverse angiospasm associated with subarachnoid hemorrhage.

The therapeutic agent, the prognosis improving drug and the antivasospastic agent of the invention can use the compound having the effect of inhibiting the function of PAR1, its pharmaceutically acceptable salt or the hydrate thereof described above by itself or as a formulation with a known pharmaceutically acceptable carrier or the like. Examples of such pharmaceutically acceptable carriers include excipients, binders, disintegrants, lubricants, colorants, flavoring agents, stabilizers, emulsifiers, absorption promoters, surfactants, pH regulators, antiseptic agents and antioxidant agents.

Moreover, the administration mode of the therapeutic agent, the prognosis improving drug and the antivasospastic agent of the invention is not particularly limited and they may be administrated orally or parenterally according to the formulations mentioned above. Examples of modes for parenteral administration include intravenous injection, drip infusion, subcutaneous injection, intradermal injection, intrathecal injection and intraperitoneal injection. Examples of formulations include tablets, powdered agents, subtle granules, granules, capsules and syrup for oral administration modes and suppositories, injectable agents, ointments and skin patches for parenteral administration modes.

When preparing an oral formulation used for oral administration mode, an excipient, and if necessary, a binder, a disintegrant, a lubricant, a colorant, a flavoring agent or the like are added to the active ingredient, and then formulated into a tablet, a coated tablet, granule, subtle granule, powdered agent, a capsule or the like by a conventional procedure.

As an excipient, for example, lactose, cornstarch, sucrose, glucose, sorbit, crystalline cellulose, silicon dioxide or the like may be used. As a binder, for example, polyvinyl alcohol, ethyl cellulose, methyl cellulose, gum arabic, hydroxypropyl cellulose, hydroxypropyl methyl cellulose or the like may be used. As a lubricant, for example, magnesium stearate, talc, silica or the like may be used. As a colorant, those that are acceptable as an additive to a pharmaceutical product may be used. As a flavoring agent, cocoa powder, menthol, aromatic acid, mint oil, camphor, cinnamon powder or the like may be used. Of course, these tablets and granules may conveniently be coated with sugar, gelatin or the like, if necessary.

According to the present invention, an injectable agent may be prepared by adding, if necessary, a nonaqueous dilution agent (e.g., glycols such as propylene glycol and polyethylene glycol, plant oils such as olive oil and alcohols such as ethanol), a suspending agent, a solubilizing agent, a stabilizer, a tonicity agent, a preservative, a pH regulator, a buffer or the like to the principal agent. Sterilization of the injectable agent may be achieved by filter sterilization, addition of a disinfectant or the like. Furthermore, the injectable agent may be produced in a form that can be prepared upon use. Specifically, the injectable agent can be made into a sterile solid composition by lyophilization or the like so as to be dissolved in sterile injectable distilled water or other solvent before use. If the agent is to be administered by transdermal absorption via an adhesive patch, it is preferable that the agent that does not form a salt, (i.e., a so-called a free form) is selected. The injectable agent may be made into a drip infusion, or an intravenous, subcutaneous or intramuscular injectable agent according to a conventional procedure.

Examples of suspending agents include methyl cellulose, Polysorbate 80, hydroxyethyl cellulose, gum arabic, powdered tragacanth, sodium carboxymethyl cellulose and polyoxyethylene sorbitan monolaurate.

Examples of solubilizing agents include polyoxyethylene hydrogenated castor oil, Polysorbate 80, nicotinic-acid amide, polyoxyethylene sorbitan monolaurate, macrogol and castor oil fatty acid ethyl ester.

Examples of stabilizers include, for example, sodium bisulfite and sodium metabisulfite. Examples of preservatives include, for example, methyl parahydroxybenzoate, ethyl parahydroxybenzoate, sorbic acid, phenol, cresol and chlorocresol.

For oral administration, an effective dose of the compound having the effect of inhibiting the function of PAR1, its pharmaceutically acceptable salt or a hydrate thereof differs depending on severity of the symptom, age, sex, weight and sensitivity difference of the patient, administration method, administration time, administration interval, administration period, property, dosage, type and type of the active ingredient of the formulation or the like, but may be determined appropriately by those skilled in the art. For example, 0.1-500 mg/day, preferably 0.5-200 mg/day, more preferably 1-100 mg/day may be orally administered to an adult (weight 60 kg).

For parenteral administration of, for example, an injectable agent, an effective amount of the compound having the effect of inhibiting the function of PAR1, its pharmaceutically acceptable salt or a hydrate thereof differs depending on severity of the symptom, age, sex, weight and sensitivity difference of the patient, administration method, administration time, administration interval, administration period, property, dosage, type and type of active ingredient of the formulation or the like, but may be determined appropriately by those skilled in the art. Such an agent dissolved or suspended in a pharmaceutically acceptable carrier such as saline or commercially available injectable distilled water can suitably be given to a patient in need of the treatment. For example, in the case of an injectable agent, 0.1-500 mg/day, preferably 0.5-200 mg/day, more preferably 1-100 mg/day may be administered to an adult (weight 60 kg).

The present invention also provides a method for treating subarachnoid hemorrhage, a method for improving prognosis of subarachnoid hemorrhage and a method for inhibiting angiospasm, comprising administering an effective amount of the compound having the effect of inhibiting the function of PAR1, its pharmaceutically acceptable salt or a hydrate thereof to a patient. According to the method of the invention, the compound having the effect of inhibiting the function of PAR1 is preferably a PAR1 antagonist, more preferably a 2-iminopyrrolidine derivative represented by Formula (I) or (III), more preferably at least one compound selected from compounds represented by Formulae (V)-(XI) and most preferably a compound represented by Formula (V). According to the method of the invention, the PAR1 inhibitor comprises its pharmaceutically acceptable salt and a hydrate thereof. According to the method of the invention, a route and a method for administering the PAR1 inhibitor are not particularly limited and may refer to the description of the pharmaceutical composition of the invention above.

Moreover, the present invention also comprises use of a 2-iminopyrrolidine derivative for producing a therapeutic agent or a prognosis improving drug for subarachnoid hemorrhage or an antivasospastic agent. According to the use of the invention, the 2-iminopyrrolidine derivative is preferably a 2-iminopyrrolidine derivative represented by Formula (I) or (III), more preferably at least one compound selected from compounds represented by Formulae (V)-(XI), and most preferably a compound represented by Formula (V). The 2-iminopyrrolidine derivative comprises its pharmaceutically acceptable salt or a hydrate thereof.

### EXAMPLES

Hereinafter, the present invention will be described specifically by way of examples which are not intended to limit the invention.

### Example 1: Production of rabbit double-hemorrhage model

As a model animal of subarachnoid hemorrhage, rabbit double-hemorrhage models were produced. The resulting rabbit double-hemorrhage models were used to find out the role of PAR1 in controlling vascular tension upon subarachnoid hemorrhage (SAH).

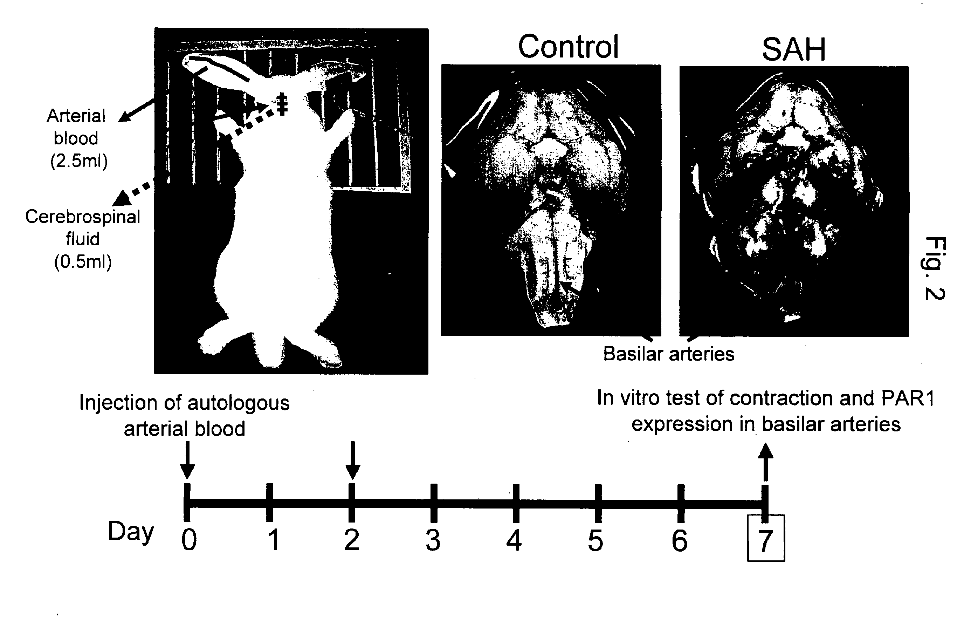

First, 2.5 ml each of autologous arterial blood was injected into the cisterna magnas twice on Days 0 and 2. Hereinafter, this group is also referred to herein as the "SAH group". Models injected with equal amounts of saline instead of autologous arterial blood constituted the control group. On Day 7, rabbits from each group were euthanized and endotheliums were removed from the dissected basilar arteries to produce ring preparations (500 µm in width) (Figure 2). The endothelium-removed basilar artery ring preparations produced in this example were used to perform experiments on contractile response in the following examples.

### Example 2: Contractions of endothelium-removed basilar arteries induced by hyperkalemic depolarization and endothelin-1

Contractions of endothelium-removed basilar arteries induced by depolarization stimulation with 118 mM K+ and 100 nM endothelin-1 (ET-1) were determined. Endothelin-1 is a substance that acts on vascular smooth muscle and cause the blood vessels to contract.

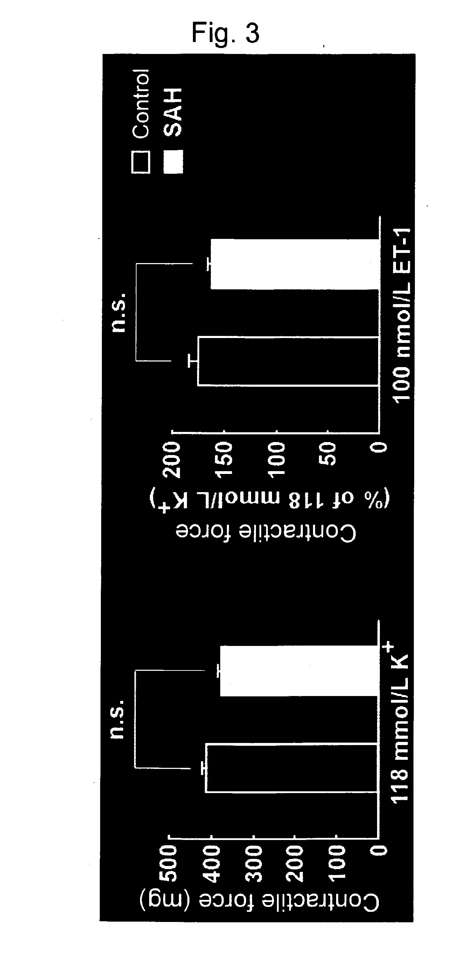

The results are shown in Figure 3. The vertical axes represent the contractile force of the ring preparations where the left graph shows contractile force upon 118 mM K+ stimulation in mg while the right graph shows contractile force upon 100 nM endothelin-1 stimulation in % where the contractile force upon 118 mM K+ stimulation is shown as 100 %. As a result, contractile response to 118 mM K+ depolarization and contractile response to endothelin-1 for the SAH group were similar to those for the control group and showed small difference therefrom.

### Example 3: Contractile responses to thrombin in rabbit basilar arteries

Contractile responses to thrombin were determined for the rabbit basilar arteries from the control group and the SAH group. Following contractile response induced by 118 mM K+ depolarization stimulation, the basilar artery ring preparations were stimulated with thrombin. Thrombin is an endogenous ligand of PAR1.

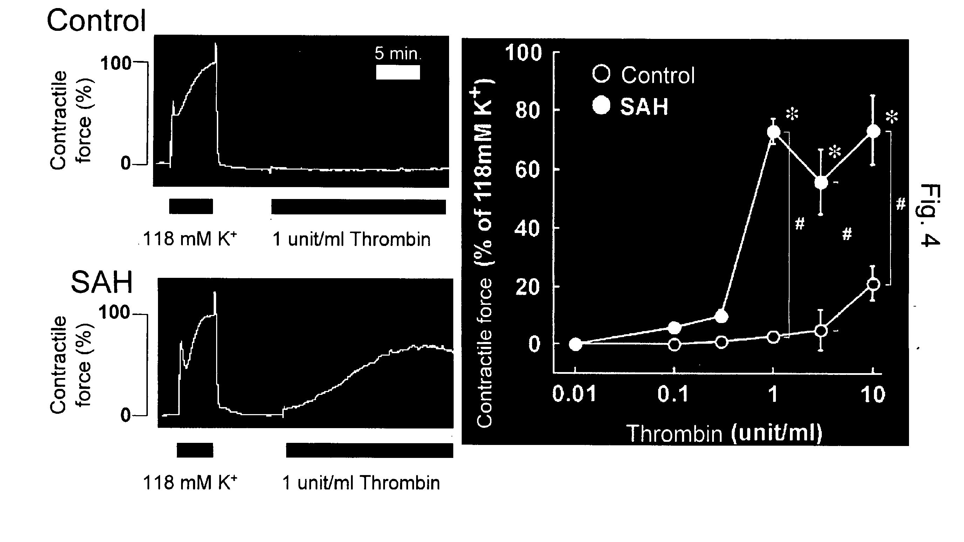

The results are shown Figure 4. The upper and lower left panels show contractile forces with time where contractile forces upon 118 mM K+ depolarization stimulation is shown as 100%. The right graph shows contractile force with respect to thrombin concentration. For the control group, thrombin (1 unit/ml) did not induce contraction and thrombin (10 units/ml) only showed mild temporary contraction (21.3 ± 1.2% of 118 mM K+-induced contraction) (Figure 4). On the other hand, for the SAH group, thrombin began to induce significant continuous contraction from 0.3 units/ml and showed contractile force of 73.1±2.8% at 1 unit/ml (Figure 4).

### Example 4: Contractile responses of rabbit basilar arteries to PAR1-AP

Same experiment as Example 3 was performed using PAR1-AP (PAR1-activating peptide) instead of thrombin.

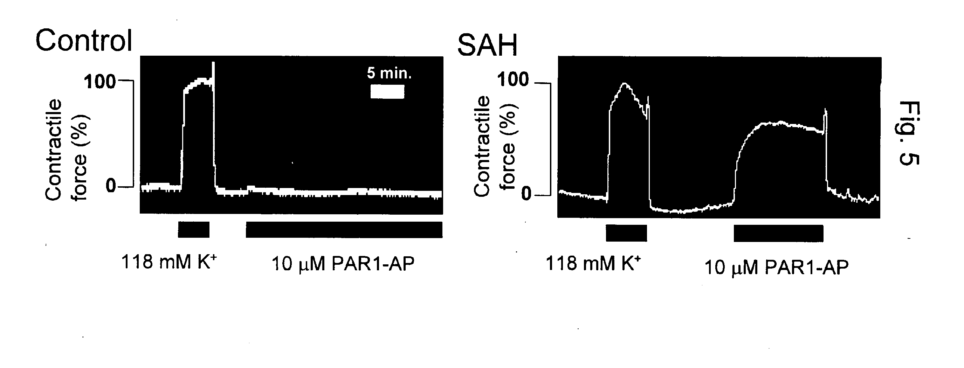

The results are shown in Figure 5. PAR1-AP (10 µM) induced contraction only for the SAH group (52.6 ±6.1 %) (Figure 5). While 100 µM of PAR1-AP induced temporary contraction for the control group, it showed increased continuous contraction for the SAH group.

### Example 5: Effect of heparinized autologous blood to inhibit increase in contractile response for SAH group

As described in Examples 3 and 4, increases in the contractile responses to thrombin and PAR1-AP were observed for the SAH group. In order to find out the effect of heparin on this increase in the contractile response, an experiment was conducted according to the method of Example 1 using models that had been injected twice with heparinized autologous blood. Heparin forms a complex with anti-thrombin III and becomes an anticoagulant that inactivates blood coagulation factor.

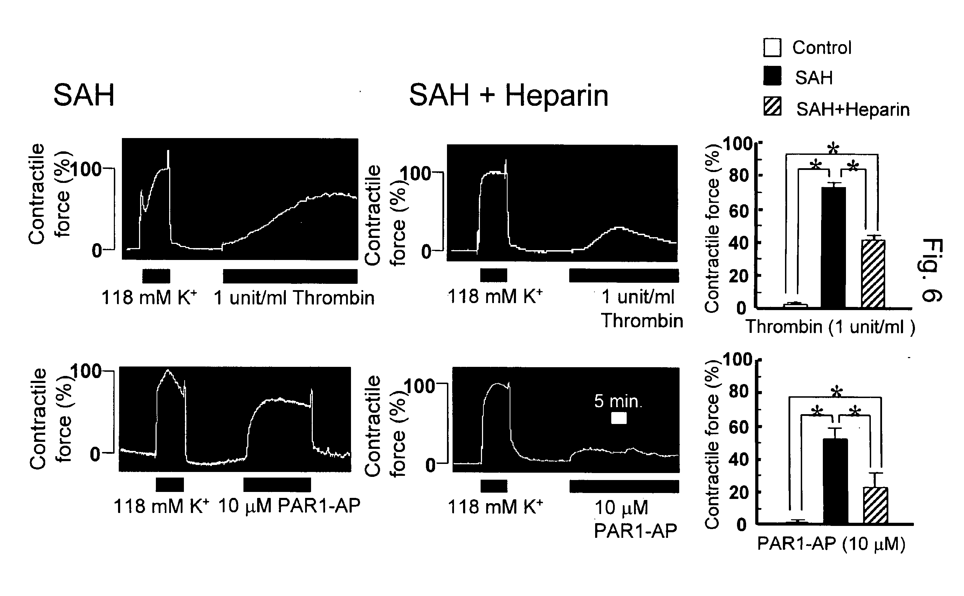

The results are shown in Figure 6. For models injected with the heparinized autologous blood, increasement in the contractile response to thrombin decreased significantly (41.6 ± 3.0% at 1 unit/ml) (Figure 6, upper panel "SAH + heparin"). In addition, increasement in the contractile response to PAR1-AP also decreased significantly (Figure 6, lower panel "SAH + heparin"). High contractions by thrombin and PAR1-AP were thus shown to be induced by the blood coagulation factor in the autologous blood which was inactivated by heparin.

### Example 6: Contractile responses of α toxin-skinned basilar arteries

### (1) Contractile responses induced by increase in Ca2+ concentration and GTPγS

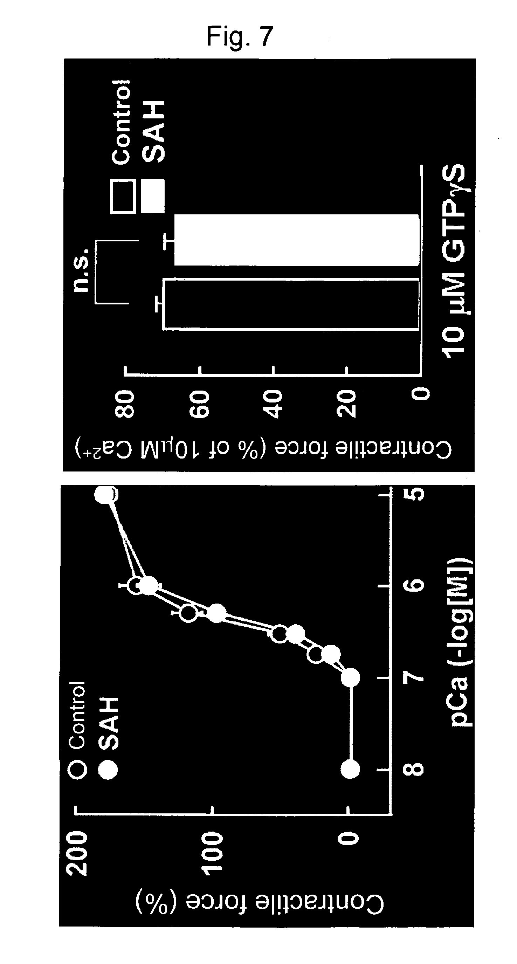

Alpha toxin is a substance that confers permeability to cell membrane. In a rabbit basilar artery that is treated with α toxin for permeabilization (hereinafter, also referred to as an "α toxin-skinned basilar artery"), contractile response associated with increase in calcium ion concentration and contractile response to GTPγS that activates G protein were measured.
The results are shown in Figure 7. The left panel in Figure 7 shows contractile force with respect to logarithm of calcium ion concentration (M) while the right panel shows contractile force upon 10 µM GTPγS stimulation (% with respect to contractile force upon 10 µM calcium ion concentration). As a result, contractile response associated with increase in the calcium concentration and contractile response associated with GTPγS addition for the α toxin-skinned basilar arteries from the SAH group were similar to those of the α toxin-skinned basilar arteries from the control group and no significant difference was observed.

### (2) Contractile responses to thrombin and PAR1-AP

Next, contractile responses of α toxin-skinned basilar arteries to thrombin and PAR1-AP were measured.

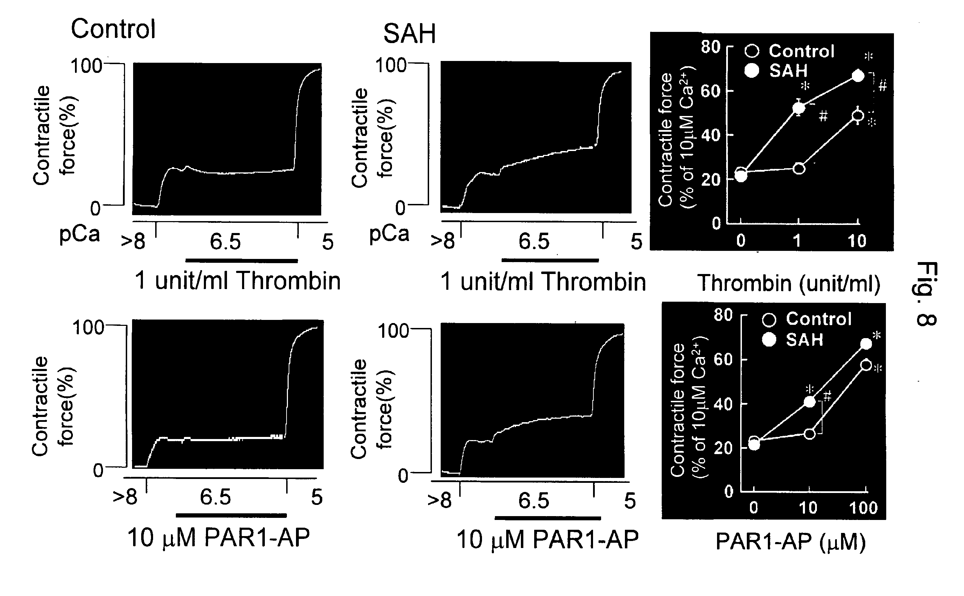

The results are shown in Figure 8. Whereas thrombin and PAR1-AP had no effect on the contractile response induced by calcium ion for the α toxin-permeabilized basilar arteries from the control group, both thrombin and PAR1-AP significantly increased the responses of the α toxin-skinned basilar arteries from the SAH group.

While sensitivities of the SAH group to calcium ion that causes contraction of the muscle and to GTPγS that activates G protein remained unchanged, sensitivities to thrombin and PAR1-AP increased.

### Example 7: Change in upregulation of PAR1 mRNA over time for SAH group

Following the first autologous blood injection, the amounts of PAR1 mRNA in the basilar arteries on Days 3, 5, 7 and 15 were examined by *in situ* hybridization.

Basilar arteries were removed to produce frozen samples. The probe used for hybridization to PAR1 mRNA was produced from cDNA of human PAR1 using *in vitro* transfer method, and labeled with digoxigenin. The samples were treated with this probe overnight. After washing off unbound probes, the samples were reacted with anti-digoxigenin antibody bound with alkaline phosphatase. Then, diaminobenzidine was used for chromogenic reaction to detect PAR1 mRNA. The developed color image was observed with a microscope, and the obtained image was analyzed by an analysis program to quantify the expression level.

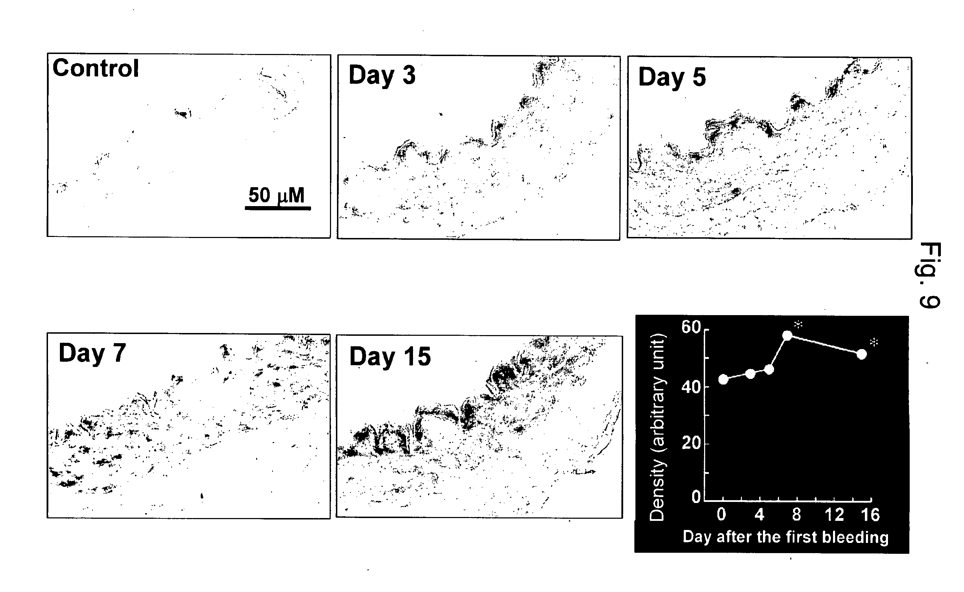

The results are shown in Figure 9. The lower right graph in Figure 9 was obtained by plotting the mRNA amounts on the days of *in situ* hybridization. As a result of the *in situ* hybridization, PAR1 mRNA in the basilar arteries from the SAH was found to be upregulated.

### Example 8: Changes in contractile response to 100 µM PAR1-AP and its duration time for SAH group

Contractile response induced by 100 µM PAR1-AP and its duration time were examined.

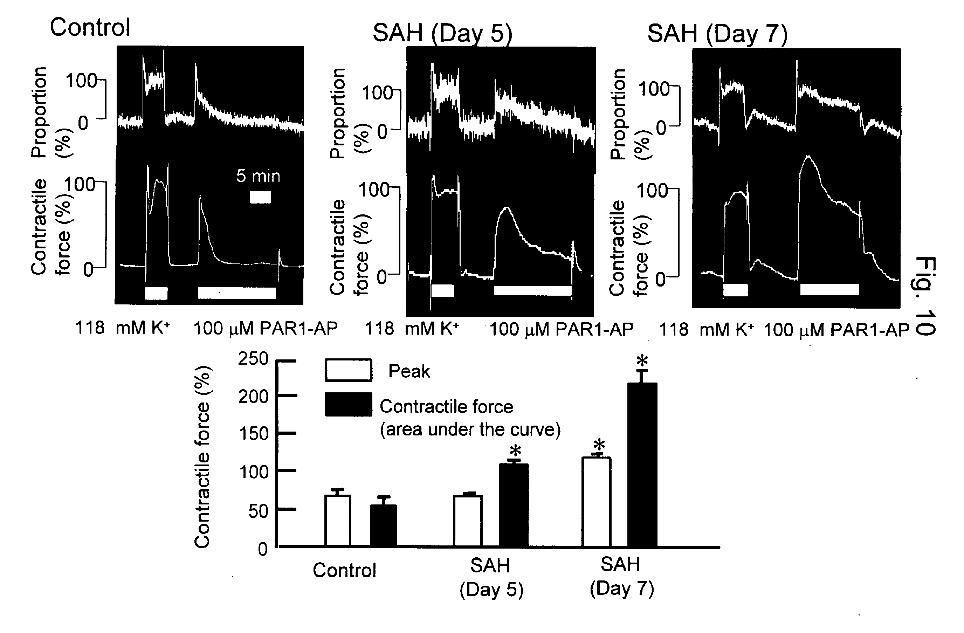

The results are shown in Figure 10. As to the contraction induced by 100 µM PAR1-AP for the SAH group, as compared to the control group, peak of the contractile force and duration of the contractile force increased 5 days after the autologous blood injection and further increased 7 days after the autologous blood injection.

Therefore, for the SAH group, PAR1 desensitization mechanism was shown to be inhibited.

### Example 9: Irreversible contraction by thrombin

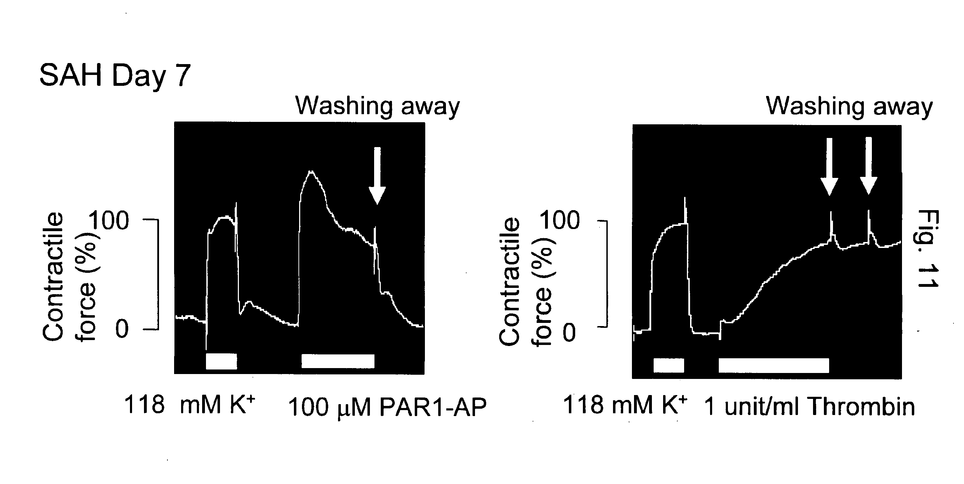

On Day 7, contractile response of SAH induced by 100 µM PAR1-AP was terminated by washing 100 µM PAR1-AP away (Figure 11, left panel). On the other hand, contractile response induced by 1 unit/ml thrombin was not changed by washing, proving irreversible contraction (Figure 11, right panel).

Thus, whereas contractile response to PAR1-AP was shown reversible, contractile response to thrombin was shown irreversible.

### Example 10: Effect of PAR1 antagonist on contractile response of rabbit basilar arteries to thrombin (1)

Contractile responses of the rabbit basilar arteries to thrombin were determined for the control group (sham surgery group), the SAH group and the SAH group coexisting with the PAR1 antagonist. Stimulation was given in the same manner as in Example 3.

In this example, the "sham surgery group (sham)" (control rabbit) refers to those obtained by injecting 3 ml saline twice into rabbit cisterns on Days 0 and 2, the "SAH group"(subarachnoid hemorrhage model) refers to those obtained by injecting 3 ml autologous blood twice into rabbit cisterns on Days 0 and 2, and the "SAH+E5555 group" (E5555-administered subarachnoid hemorrhage model) refers to those obtained by injecting 3ml autologous blood supplemented with 600 µg E5555 twice into rabbit cisterns on Days 0 and 2.

PAR1 antagonist used in this example is E5555 represented by Formula (V) (WO 02/085855, pamphlet).

On Day 7, the basilar arteries were removed to assess contractile responses to 118 mM K+ and 1 unit/ml thrombin.

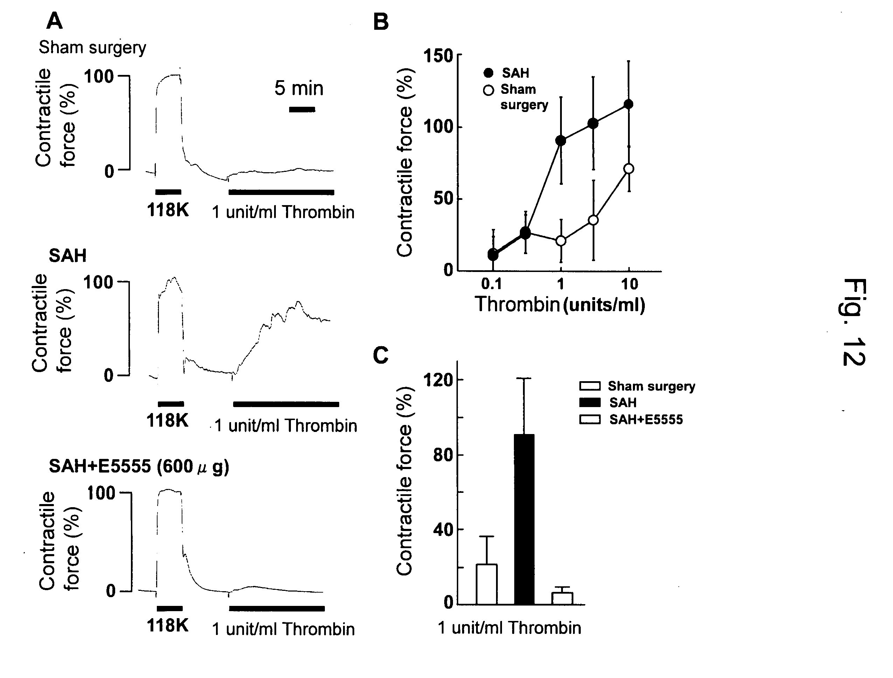

The results are shown in Figure 12. Figure 12 shows representative records of actual contractile responses.

Figure 12A shows contractile responses of the basilar arteries to thrombin for the sham operation group, the SAH group and the SAH + E5555 group from the top. For the sham surgery group, 1 unit/ml thrombin caused slight contraction of the removed basilar arteries. For the SAH, a higher level of contractile response was observed with 1 unit/ml thrombin stimulation. For the subarachnoid hemorrhage model co-administered with E5555 (SAH+E5555), contraction by 1 unit/ml thrombin was small.

Figure 12B shows the curves of contractile responses induced by thrombin with respect to dose thereof for the SAH group and the sham surgery group. As a result, contractile response induced by thrombin was enhanced for the basilar arteries from the subarachnoid hemorrhage model.

Figure 12C shows an effect of E5555 to prevent excessive contractile response to thrombin caused by subarachnoid hemorrhage. Contractile responses to 1 unit/ml thrombin were compared among the sham surgery group, the SAH group and the SAH + E5555 group. The results are shown in average value ± standard error (n=3). As a result, co-administration of E5555 and autologous blood significantly suppressed the increased reactivity to thrombin caused by subarachnoid hemorrhage.

These results show that PAR1 is involved in induction of high contractile property by autologous blood as well as impairment of desensitization, and that this induction of high contractile property and impairment of desensitization can be suppressed with a PAR1 antagonist. PAR1 was clearly found to be involved in both induction of high contractile property following bleeding and angiospasm. Specifically, a PAR1 inhibitor not only regresses cerebral vasospasm following subarachnoid hemorrhage but also possibly prevent the occurrence thereof, suggesting itself as a therapeutic agent for subarachnoid hemorrhage with a new action mechanism.

### Example 11: Effect of PAR 1 antagonist on contractile response of rabbit basilar arteries to thrombin (2)

In the same manner as Example 10, contractile responses of the rabbit basilar arteries to thrombin were determined for the control group (the sham surgery group), the SAH group and the SAH group coexisting with the PAR1 antagonist.

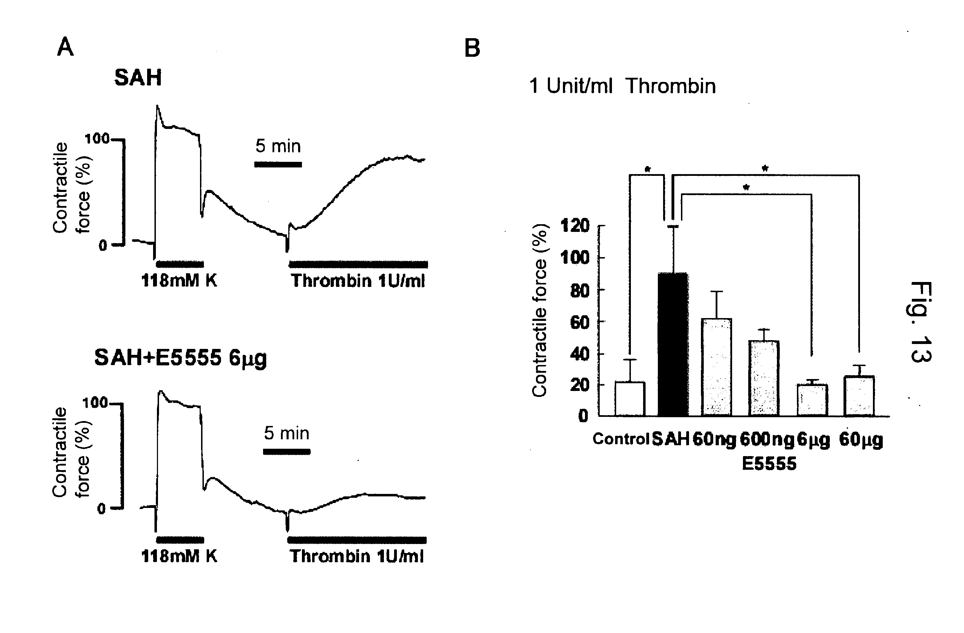

In this example, 3 ml each of autologous blood supplemented with 60 ng, 600 ng, 6 µg or 60 µg of E5555 was injected twice into rabbit cistern on Days 0 and 2. Representative records of actual contractile responses are shown in Figure 13.

Figure 13A shows contractile responses of the basilar arteries to thrombin for the SAH group and the SAH+E5555 (6 µg) group from the top. For the subarachnoid hemorrhage models given E5555 and autologous blood at the same time (SAH+E5555), contraction by 1 unit/ml thrombin was smaller than that for the SAH group.

Figure 13B shows, dose-dependent effect of E5555 to prevent excessive contractile response to thrombin caused by subarachnoid hemorrhage. Contractile responses to 1 unit/ml thrombin were compared between the sham surgery group (control) and the SAH+E5555 group. The results are shown in average value ± standard error. As a result, co-administration of autologous blood and E5555 suppressed the increased reactivity of thrombin caused by subarachnoid hemorrhage with 60 or higher ng of E5555 in a dose-dependent manner. In particular, contractile responses were significantly suppressed for the SAH+E5050 (6 µg) group and the SAH+E5050 (60 µg) group as compared to the SAH group.

Example 11 demonstrates that E5555 works at lower dose than the dose shown in Example 10 for excessive contractile response to thrombin caused by subarachnoid hemorrhage.

Thus, thrombin induces high contractile response in the rabbit double-hemorrhage model, and this high contractile response seems to result from PAR1 upregulation and impaired desensitization of the receptor. For SAH, activation of thrombin as an endogenous agonist of PAR1 seems to be a key action for the angiospasm expression following bleeding.

Moreover, since high contractile property of the basilar artery by increased PAR1 was suppressed by the PAR1 inhibitor, the PAR1 inhibitor can be effective as a therapeutic agent and a prognosis improving drug for subarachnoid hemorrhage and an antivasospastic agent.

### INDUSTRIAL APPLICATION

The present invention provides a therapeutic agent for subarachnoid hemorrhage, a drug for improving prognosis of subarachnoid hemorrhage and an antivasospastic agent which comprise a compound having an effect of inhibiting the function of PAR1 as an active ingredient. In the event of subarachnoid hemorrhage, the function of PAR1 is increased, as a result of which high contraction of the basilar artery is induced. Thus, since a compound having an effect of inhibiting the function of PAR1 can suppress high contraction of the basilar artery, it is useful as an active ingredient in the therapeutic agent and the prognosis improving drug for subarachnoid hemorrhage and the antivasospastic agent.

## Claims

1. A therapeutic agent for subarachnoid hemorrhage or a drug for improving prognosis of subarachnoid hemorrhage, comprising a compound having an effect of inhibiting the function of protease-activated receptor-1, its pharmaceutically acceptable salt or a hydrate thereof.

2. A therapeutic agent or an improving drug according to Claim 1, wherein the compound having the effect of inhibiting the function of protease-activated receptor-1 is an antagonist of protease-activated receptor-1.

3. A therapeutic agent or an improving drug according to either one of Claims 1 and 2, wherein the compound having the effect of inhibiting the function of protease-activated receptor-1 is 2-iminopyrrolidine derivative.

4. A therapeutic agent for subarachnoid hemorrhage or a drug for improving prognosis of subarachnoid hemorrhage comprising a 2-iminopyrrolidine derivative, wherein the 2-iminopyrrolidine derivative is a compound represented by Formula (I) 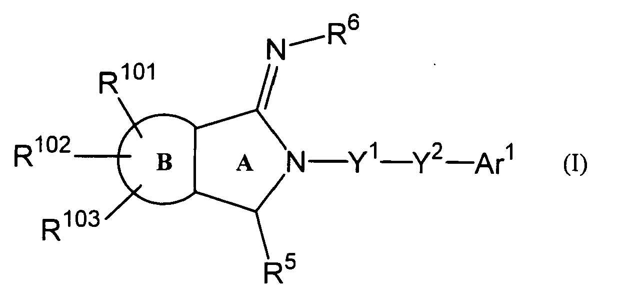 [wherein, ring A indicates a pyrrolidine ring; ring B indicates a benzene ring or a pyridine ring; R¹⁰¹, R¹⁰² and R¹⁰³ independently indicate, identically or differently, a hydrogen atom, a halogen atom, C₁-C₆ alkyl group or C₁-C₆ alkoxy group; R⁵ indicates a hydrogen atom, C₁-C₆ alkyl group or C₁-C₆ alkoxy C₁-C₆ alkyl group; R⁶ indicates a hydrogen atom, C₁-C₆ alkyl group or C₁-C₆ alkyloxy carbonyl group; Y¹ indicates a single bond or -CH₂-; Y² indicates a single bond or -CO-; Ar¹ indicates a hydrogen atom or a group represented by Formula (II) 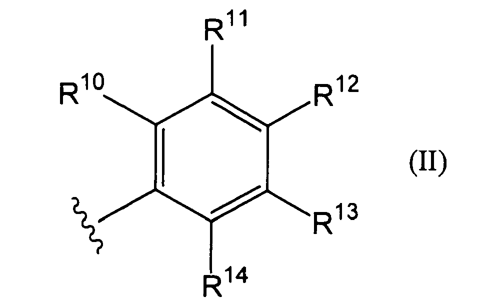 (wherein, R¹⁰, R¹¹, R¹², R¹³ and R¹⁴ independently indicate, identically or differently, a hydrogen atom, C₁-C₆ alkyl group, hydroxyl group, C₁-C₆ alkoxy group, morpholinyl group, piperazinyl group which may or may not have a substituent, piperidinyl group which may or may not have a substituent or pyrrolidinyl group which may or may not have a substituent, and R¹¹ and R¹² or R¹² and R¹³ may bind to each other to form a 5-8-membered heterocycle)],
its pharmaceutically acceptable salt, or a hydrate thereof.

5. A therapeutic agent for subarachnoid hemorrhage or a drug for improving prognosis of subarachnoid hemorrhage comprising a 2-iminopyrrolidine derivative, wherein the 2-iminopyrrolidine derivative is a compound represented by Formula (III) 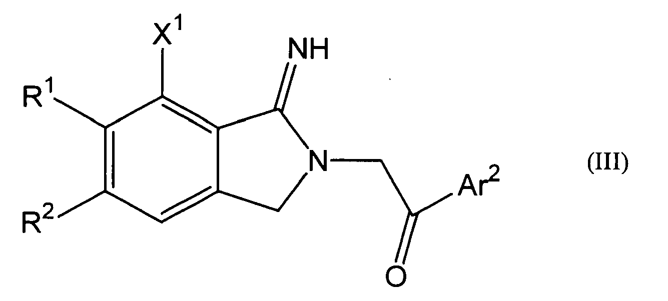 [wherein, R¹ and R² independently indicate, identically or differently, a hydrogen atom, methoxy group or ethoxy group; X¹ indicates a hydrogen atom or a halogen atom; Ar² indicates methyl group, ethyl group, methoxy group, ethoxy group, t-butyl group, morpholinyl group or phenyl group which may be substituted with one or more substituents selected from substituents represented by the following Formula (IV), 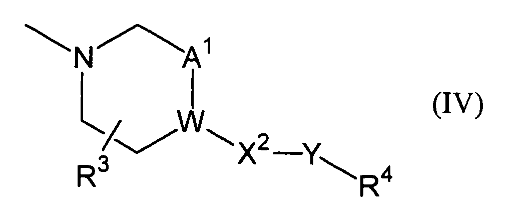 wherein, W indicates -CH- or a nitrogen atom; A¹ indicates -CH₂- or a single bond; R³ indicates a hydrogen atom or -OR 5^{a}; X² indicates -CH₂-, an oxygen atom, a single bond or carbonyl group; Y indicates a single bond or C₁-C₄ alkylene group; R⁴ indicates a hydrogen atom, -OR^{6a}, cyano group or -COOR⁷; R^{5a}, R^{6a} and R⁷ independently indicate, identically or differently, a hydrogen atom or C₁-C₄ alkyl group],
its pharmaceutically acceptable salt or a hydrate thereof.

6. A therapeutic agent or an improving drug according to Claim 5 wherein R¹ and R² are ethoxy groups while X¹ is a fluorine atom.

7. A therapeutic agent for subarachnoid hemorrhage or a drug for improving prognosis of subarachnoid hemorrhage comprising a 2-iminopyrrolidine derivative, wherein the 2-iminopyrrolidine derivative is one selected from the group consisting of compounds represented by Formulae (V)-(XI), its pharmaceutically acceptable salt or a hydrate thereof.

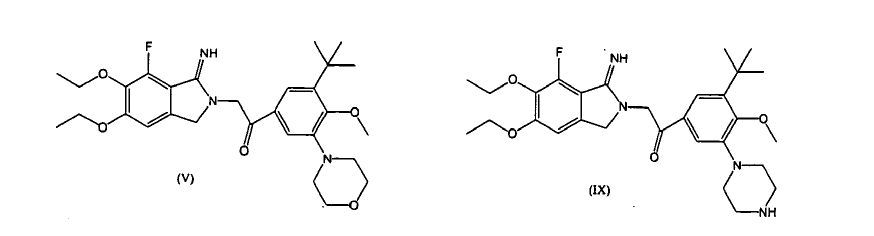

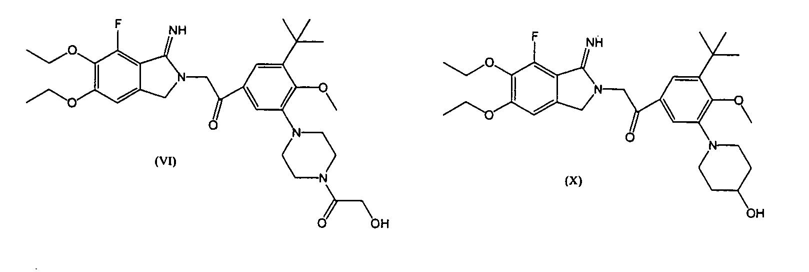

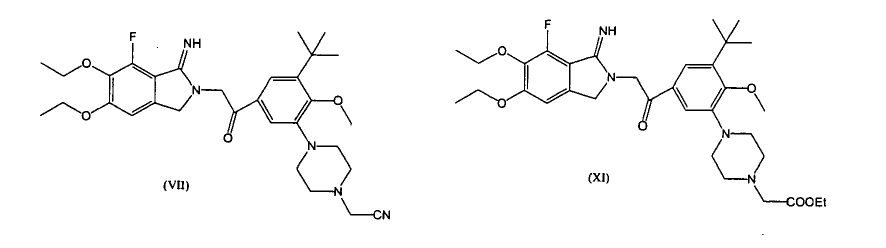

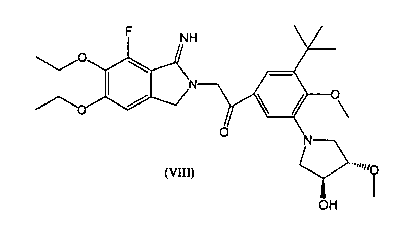

8. A therapeutic agent for subarachnoid hemorrhage or a drug for improving prognosis of subarachnoid hemorrhage comprising a 2-iminopyrrolidine derivative, wherein the 2-iminopyrrolidine derivative is a compound represented by Formula (V), its pharmaceutically acceptable salt or a hydrate thereof.

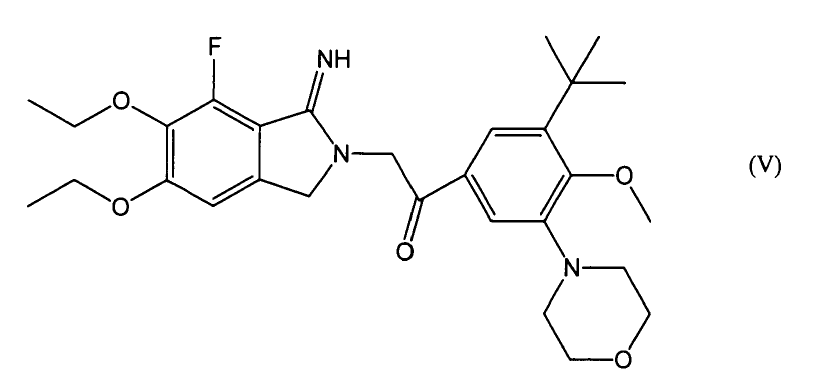

9. An antivasospastic agent comprising a compound having an effect of inhibiting the function of protease-activated receptor-1, its pharmaceutically acceptable salt or a hydrate thereof.

10. An antivasospastic agent according to Claim 9, wherein the compound having the effect of inhibiting the function of protease-activated receptor-1 is an antagonist of protease-activated receptor-1.

11. An antivasospastic agent according to either one of Claims 9 and 10 wherein the compound having the effect of inhibiting the function of protease-activated receptor-1 is a 2-iminopyrrolidine derivative.

12. An antivasospastic agent comprising a 2-iminopyrrolidine derivative, wherein the 2-iminopyrrolidine derivative is a compound represented by Formula (I) 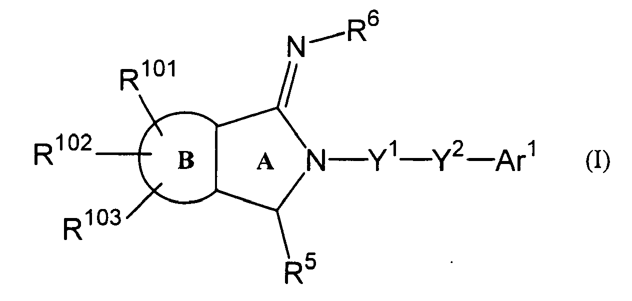 [wherein, ring A indicates a pyrrolidine ring; ring B indicates a benzene ring or a pyridine ring; R¹⁰¹, R¹⁰² and R¹⁰³ independently indicate, identically or differently, a hydrogen atom, a halogen atom, C₁-C₆ alkyl group or C₁-C₆ alkoxy group; R⁵ indicates a hydrogen atom, C₁-C₆ alkyl group or C₁-C₆ alkoxy C₁-C₆ alkyl group; R⁶ indicates a hydrogen atom, C₁-C₆ alkyl group or C₁-C₆ alkyloxy carbonyl group; Y¹ indicates a single bond or -CH₂-; Y² indicates a single bond or -CO-; Ar¹ indicates a hydrogen atom or a group represented by Formula (II) 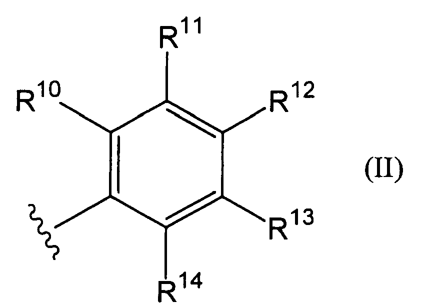 (wherein, R¹⁰, R¹¹, R¹², R¹³ and R¹⁴ independently indicate, identically or differently, a hydrogen atom, C₁-C₆ alkyl group, hydroxyl group, C₁-C₆ alkoxy group, morpholinyl group, piperazinyl group which may or may not have a substituent, piperidinyl group which may or may not have a substituent or pyrrolidinyl group which may or may not have a substituent, and R¹¹ and R¹² or R¹² and R¹³ may bind to each other to form a 5-8-membered heterocycle)],
its pharmaceutically acceptable salt or a hydrate thereof.

13. An antivasospastic agent comprising a 2-iminopyrrolidine derivative, wherein the 2-iminopyrrolidine derivative is a compound represented by Formula (III) 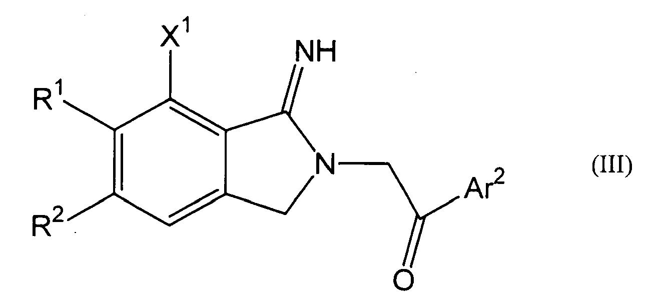 [wherein, R¹ and R² independently indicate, identically or differently, a hydrogen atom, methoxy group or ethoxy group; X¹ indicates a hydrogen atom or a halogen atom; Ar² indicates methyl group, ethyl group, methoxy group, ethoxy group, t-butyl group, morpholinyl group or phenyl group which may be substituted with one or more substituents selected from substituents represented by the following Formula (IV), 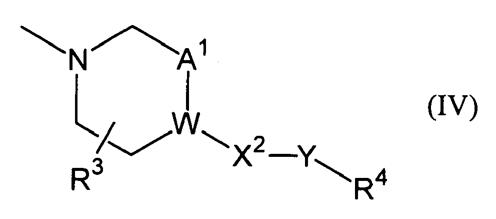 (wherein, W indicates -CH- or a nitrogen atom; A¹ indicates -CH₂- or a single bond; R³ indicates a hydrogen atom or -OR^{5a}; X² indicates -CH₂-, an oxygen atom, a single bond or carbonyl group; Y indicates a single bond or C₁-C₄ alkylene group; R⁴ indicates a hydrogen atom, -OR^{6a}, cyano group or -COOR⁷; R^{5a}, R^{6a} and R⁷ independently indicate, identically or differently, a hydrogen atom or C₁-C₄ alkyl group)],
its pharmaceutically acceptable salt or a hydrate thereof.

14. An antivasospastic agent according to Claim 13, wherein R¹and R² are ethoxy groups while X¹ is a fluorine atom.

15. An antivasospastic agent comprising a 2-iminopyrrolidine derivative, wherein the 2-iminopyrrolidine derivative is any one selected from the group consisting of compounds represented by Formulae (V)-(XI), its pharmaceutically acceptable salt or a hydrate thereof.

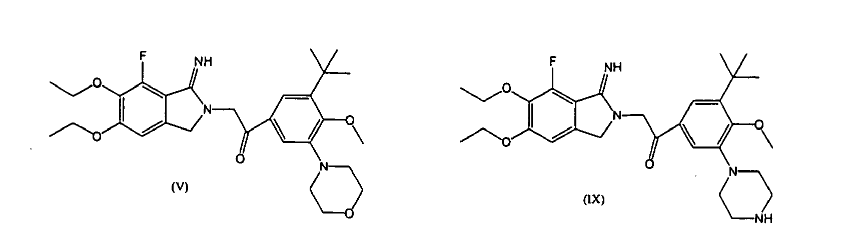

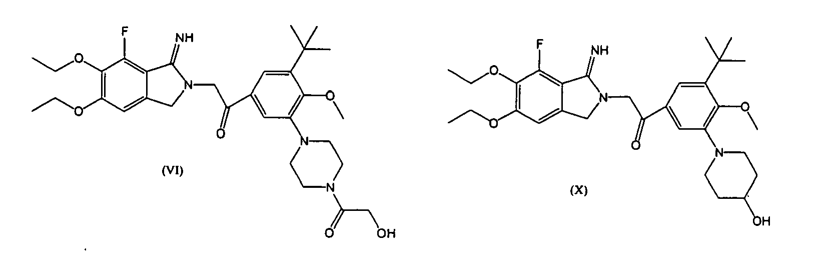

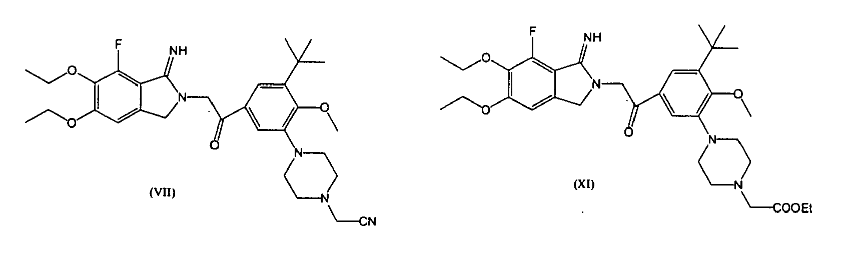

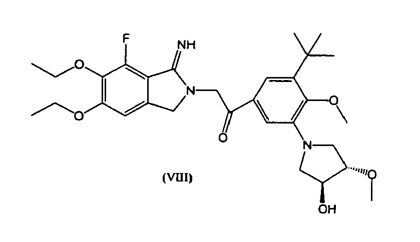

16. An antivasospastic agent comprising a 2-iminopyrrolidine derivative, wherein the 2-iminopyrrolidine derivative is a compound represented by Formula (V), its pharmaceutically acceptable salt or a hydrate thereof.

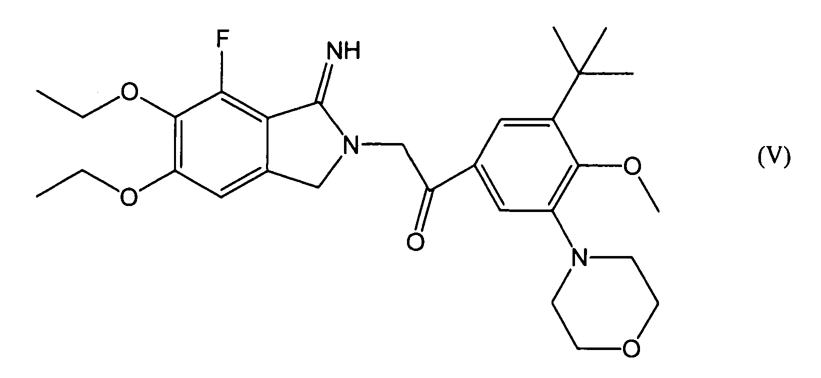

17. A method for treating subarachnoid hemorrhage or a method for improving prognosis of subarachnoid hemorrhage, comprising administering an effective amount of a compound having an effect of inhibiting the function of protease-activated receptor-1, its pharmaceutically acceptable salt or a hydrate thereof to a patient.

18. A method for treating subarachnoid hemorrhage or a method for improving prognosis of subarachnoid hemorrhage, comprising administering an effective amount of the 2-iminopyrrolidine derivative according to at least one of Claims 4-8 to a patient.

19. A method for preventing angiospasm, comprising administering an effective amount of a compound having an effect of inhibiting the function of protease-activated receptor-1, its pharmaceutically acceptable salt or a hydrate thereof to a patient.

20. A method for preventing angiospasm, comprising administering an effective amount of the 2-iminopyrrolidine derivative according to at least one of Claims 12-16 to a patient.

21. Use of the 2-iminopyrrolidine derivative according to at least one of Claims 4-8 for producing a therapeutic agent for subarachnoid hemorrhage or a drug for improving prognosis of subarachnoid hemorrhage.

22. Use of the 2-iminopyrrolidine derivative according to at least one of Claims 12-16 for producing an antivasospastic agent.


SEQUENCE LISTING

<110> EISAI R & D Management Co., Ltd.
Kyushu University, National University Corporation

<120> Remedy for Angiospasm Accompanying Subarachnoid Hemorrhage containing
Thrombin Receptor Antagonist as the Active Ingredient

<130> G06-0123

<150> US 60/626,412
<151> 2004-11-09

<150> PCT/JP2005/005068
<151> 2005-03-15

<160> 2

<170> PatentIn version 3.3

<210> 1
<211> 6
<212> PRT
<213> Homo sapiens

<400> 1

Ser Phe Leu Leu Arg Asn
1               5

<210> 2
<211> 6
<212> PRT
<213> Frog

<400> 2

Thr Phe Arg Ile Phe Asp
1               5